# FASCICULE DE BREVET EUROPEEN

(11) **EP 4 376 973 B1**
(45) Date de publication et mention de la délivrance du brevet: **02.07.2025**
(21) Numéro de dépôt: 22757294.8
(22) Date de dépôt: 28.07.2022
(51) Int. Cl.: G01N 33/18, G01N 33/02, B01D 15/38, B01J 20/26, C08F 226/06, C08F 222/10

(54) **POLYMÈRE À EMPREINTES MOLÉCULAIRES, ET SON UTILISATION DANS LA QUANTIFICATION D'UN PESTICIDE DANS UN ÉCHANTILLON**
MOLEKULAR GEPRÄGTES POLYMER, UND SEINE VERWENDUNG ZUR QUANTIFIZIERUNG EINES PESTIZIDS IN EINER PROBE
MOLECULARLY IMPRINTED POLYMER, AND ITS USE IN THE QUANTIFICATION OF A PESTICIDE IN A SAMPLE

(30) Priorité: 30.07.2021 FR 2108363
(43) Date de publication de la demande: 05.06.2024
(73) Titulaire: Ecole Supérieure de Physique et de Chimie Industrielles de la Ville de Paris, 75005 Paris (FR); PARIS SCIENCES ET LETTRES, 75006 Paris (FR); Centre National de la Recherche Scientifique, 75016 Paris (FR); Agence nationale de sécurité sanitaire de l'alimentation,de l'environnement et du travail, 94700 Maisons-Alfort (FR); SORBONNE UNIVERSITE, 75006 Paris (FR)
(72) Inventeur: PICHON, Valérie, 92100 BOULOGNE-BILLANCOURT (FR); COMBES, Audrey, 91120 PALAISEAU (FR); BOSMAN, Pauline, 94700 MAISONS-ALFOR (FR); LAVISON-BOMPARD, Gwenaelle, 92140 CLAMART (FR); LAMBERT, Marine, 94320 THIAIS (FR)
(74) Mandataire: Cabinet Grosset-Fournier & Demachy
(86) Numéro de dépôt international: PCT/EP2022/071238
(87) Numéro de publication internationale: WO 2023/006895

(56) Documents cités:
- CN-A- 110 117 342
- CN-A- 112 694 564
- US-A1- 2012 052 757
- DONOGHUE J K ; ET AL: "Determination of Dissolved Kepone by Direct Addition of XAD-2 Resin to Water", ANAL. CHEM. AT. ENERGY COMM. REP. ANAL. CHEM. M. ANAL. CHEM.EML PROCEDURES MANUAL VAN ZANTEN, B.; LELIART, G. ANAL. CHEM.. ANAL. CHEM. T. M. HEALTH PHYS, 1 January 1980 (1980-01-01), pages 779 - 780, XP055904067, Retrieved from the Internet <URL:https://pubs.acs.org/doi/abs/10.1021/ac50054a044> [retrieved on 20220322]

## Description

La présente invention concerne un polymère à empreintes moléculaires, et son utilisation dans la quantification d'un pesticide dans un échantillon. L'invention concerne également une méthode de préparation dudit polymère.

Le chlordécone (CLD) est un pesticide organochloré persistant inclus dans la convention de Stockholm de 2009 qui interdit ou limite l'utilisation de 21 pesticides et produits dangereux. Il a été largement utilisé dans les Antilles françaises à partir de 1972 pour lutter contre le charançon du bananier. Il a été interdit en 1990 mais utilisé sous dérogation jusqu'en 1993. Cependant, en raison de sa persistance et de par son utilisation intensive, il est toujours retrouvé dans les sols antillais, et dans les eaux fluviales et souterraines et les milieux marins. Le chlordécone est métabolisé dans le foie chez l'homme par le chlordécone réductase pour donner le chlordécol (CLDOH).

Au cours du temps, des méthodes de dosage du chlordécone et du chlordécol ont été développées. Cependant, ces méthodes manquent de sélectivité, du fait de la présence d'autres composants dans les échantillons à analyser, pouvant interférer dans l'analyse.

Il existe donc un besoin de disposer de moyens de pouvoir purifier un échantillon, afin d'assurer la sélectivité de l'analyse, et de pouvoir quantifier les pesticides de façon fiable et répétable.

Récemment, des polymères à empreintes moléculaires (MIP) ont été développés pour le traitement des échantillons avant analyse. Il s'agit de polymères ayant été spécialement conçus pour avoir une affinité pour le produit à analyser. Pour ce faire, le polymère a été fabriqué en présence de la molécule d'intérêt ou d'un analogue structural, de sorte que dans la structure 3D dudit polymère, il existe des sites pouvant accueillir ladite molécule et la retenir (US 2012/052757 A1, CN 110117342 A et CN 112694564 A).

Cependant, ces polymères sont difficiles à concevoir car le succès de cette approche dépend des conditions de polymérisation très spécifiques, notamment par rapport au choix des monomères, de l'agent réticulant, et du porogène. De ce fait, et à ce jour, aucun polymère pouvant efficacement retenir le chlordécone et/ou le chlordécol n'a été préparé, malgré l'intérêt évident qu'un tel polymère représente.

### CONTEXTE DE L'INVENTION

L'un des buts de l'invention est de fournir un polymère à empreintes moléculaires.

Un autre but de la présente invention est une méthode de préparation d'un polymère à empreintes moléculaires.

Un autre but de l'invention est de pouvoir utiliser ledit polymère à empreintes moléculaires dans la détection et/ou la quantification d'un pesticide.

Encore un autre but de l'invention est de fournir une méthode de préparation de l'échantillon en amont de l'analyse pour la détection et/ou la quantification d'un pesticide dans des milieux divers, comme par exemple du sérum.

Les inventeurs ont entamé une recherche approfondie pour mettre au point un polymère à empreintes moléculaires ayant des propriétés de rétention du chlordécone et/ou du chlordécol adéquate, et pouvant être utilisé dans l'analyse d'échantillons vis à vis de ce pesticide et /ou son métabolite principal.

**Un premier objet** de la présente invention est un polymère à empreintes moléculaires (MIP) dans lequel le polymère est du poly-4-vinylpyridine, ou du poly-2-vinylpyridine, réticulé par un agent réticulant,
dans lequel l'agent réticulant est choisi parmi l'éthylène glycol diméthacrylate (EGDMA), le triméthylolpropane triméthacrylate (TRIM) et le pentaérythritol triacrylate (PETRA), ledit polymère à empreintes moléculaires contenant des sites d'empreinte pour du chlordécol et/ou du chlordécone.

La présente invention concerne un polymère à empreintes moléculaires (MIP) dans lequel le polymère est du poly-4-vinylpyridine, ou du poly-2-vinylpyridine, réticulé par un agent réticulant,
dans lequel l'agent réticulant est choisi parmi l'éthylène glycol diméthacrylate (EGDMA), le triméthylolpropane triméthacrylate (TRIM) et le pentaérythritol triacrylate (PETRA),
ledit polymère à empreintes moléculaires contenant des sites d'empreinte pour du chlordécol et/ou du chlordécone,
notamment dans lequel le polymère est du poly-4-vinylpyridine réticulé par de l'éthylène glycol diméthacrylate (EGDMA).

Par *« polymère à empreintes moléculaires »* on entend, au sens de la présente invention, un polymère synthétique, comprenant des sites récepteurs pouvant accueillir une molécule organique, à savoir le chlordécone et/ou le chlordécol.

La structure chimique des sites récepteurs permet des interactions avec les molécules cibles d'avoir lieu, telles que des interactions de type van der Waals, non-covalentes.

Ainsi, le polymère à empreintes moléculaires peut « *capter* » la molécule cible de manière réversible, et sélective, par rapport à d'autres composants, ou « *interférents* », pouvant être présents dans l'échantillon à analyser.

Parmi les interférents, on peut citer notamment d'autres classes de pesticides, des composés principaux de la matrice (protéines pour les denrées carnées ou le sérum, les pigments pour les denrées d'origine végétales, les sels pour les urines).

Par « *réversible »* on entend que la molécule cible est retenue par le polymère à empreintes moléculaires, par des liaisons non-covalentes, et peut être éliminée sans modification chimique de ladite molécule cible, et dudit polymère à empreintes moléculaires. L'élimination étant effectuée par l'utilisation d'un solvant apte à rompre les interactions non-covalentes entre la molécule cible et le polymère à empreintes moléculaires.

Par « *sélective* » on entend que le polymère à empreintes moléculaires a une meilleure affinité pour la molécule cible par rapport à d'autres composants pouvant être contenus dans l'échantillon à analyser.

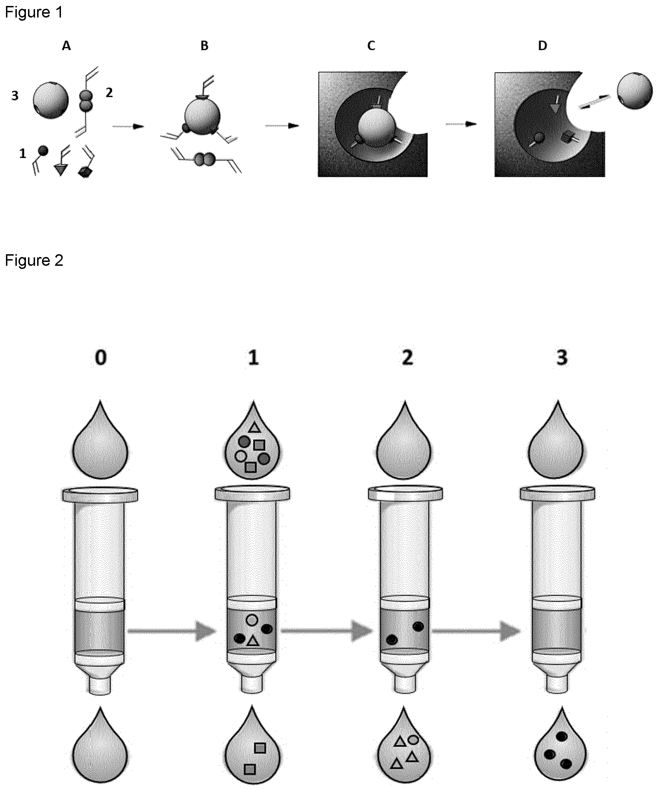

Par « *sites d'empreinte »* on entend les sites récepteurs susmentionnés, schématiquement représentés dans les **Figures 1C** et **1D****.** Les sites d'empreinte, dans le polymère à empreintes moléculaires selon la présente invention, sont des sites disponibles pour accueillir le chlordécone et/ou du chlordécol, et ne contiennent donc pas déjà lesdites molécules. Il s'agit donc, sauf indication contraire, d'un polymère à empreintes moléculaires dépourvu de chlordécone et/ou de chlordécol.

Le 4-vinylpyridine (4-VP) et le 2-vinylpyridine (2-VP) sont des monomères pouvant subir une polymérisation radicalaire pour conduire au poly-4-vinylpyridine et au poly-2-vinylpyridine respectivement. Les structures des deux monomères sont représentées ci-dessous :

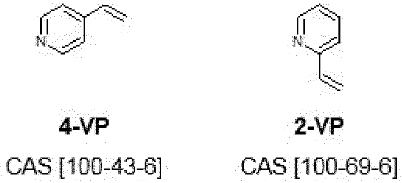

Les agents de réticulation utilisés dans la présente invention présentent des motifs de type acrylate et ont les structures suivantes :

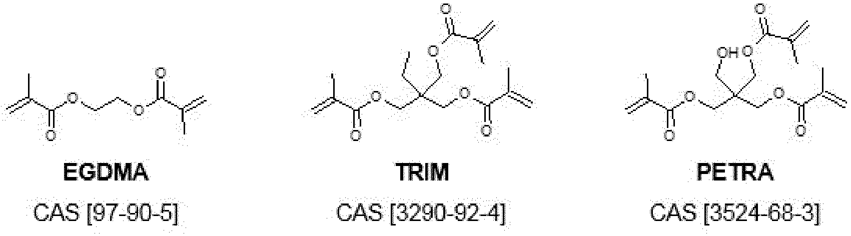

Au sens de la présente invention on entend par *« polymère réticulé »* le fait que le monomère et l'agent réticulant ont été copolymérisés.

Le chlordécone, commercialisé dans les formulations Kepone^{®}, Curlone^{®} ou Merex^{®}, est une molécule polychloré dont le nom IUPAC est le *« 1,2,3,4,6,7,8,9,10,10-decarchloropenta-cyclo[5.3.0.0^{2,6}.0^{3,9}.0^{4,8}]decan-5-one ».*

Le chlordécol, ou *« 1,2,3,4,6,7,8,9, 10, 10-decachloro-pentacyclo[5.3.0.0^{2,6}.0^{3,9}.0^{4,8}]decan-5-*ol » est un composé de structure similaire à celle du chlordécone, mais présentant une fonction hydroxyle au lieu d'une fonction cétone.

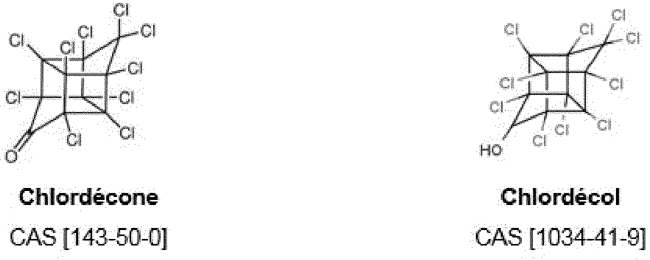

Selon un mode de réalisation particulier, la présente invention concerne un polymère à empreintes moléculaires (MIP) dans lequel le polymère est du poly-4-vinylpyridine (4 VP MIP), réticulé par un agent réticulant,
dans lequel l'agent réticulant est choisi parmi l'éthylène glycol diméthacrylate (EGDMA), le triméthylolpropane triméthacrylate (TRIM) et le pentaérythritol triacrylate (PETRA),
ledit polymère à empreintes moléculaires contenant des sites d'empreinte pour du chlordécol et/ou du chlordécone.

Selon un mode de réalisation particulier, la présente invention concerne un polymère à empreintes moléculaires tel que défini ci-dessus, dans lequel le polymère est du poly-4-vinylpyridine réticulé par de l'éthylène glycol diméthacrylate (EGDMA).

Selon un autre mode de réalisation particulier, la présente invention concerne un polymère à empreintes moléculaires tel que défini ci-dessus, imprimé par du chlordécol.

Selon ce mode de réalisation particulier, les sites d'empreinte sont formés à partir du chlordécol. Les Inventeurs ont trouvé de façon surprenante et démontré que le polymère à empreintes moléculaires ainsi formé est capable de retenir non seulement du chlordécol utilisé dans sa préparation, mais également le chlordécone, malgré le fait que le chlordécone ne possède pas la fonction hydroxyle du chlordécol.

Selon un autre mode de réalisation particulier, la présente invention concerne un polymère à empreintes moléculaires tel que défini ci-dessus, ledit polymère à empreintes moléculaires possédant une capacité de retenir du chlordécone à raison de 36 µg par gramme de polymère. Dans ce qui suit, le composé à retenir, à savoir le chlordécone et/ou le chlordécol, est également appelé « *analyte* ».

Dans ce mode de réalisation, une solution de chlordécone, notamment à une concentration allant jusqu'à 36 µg/ml, est percolée à travers une cartouche comprenant 1 gramme de polymère à empreintes moléculaires. Ainsi, 36 µg de chlordécone peuvent être retenus sur la cartouche, par gramme de polymère. Cette propriété de rétention prouve la présence des sites d'empreinte et peut être utilisée comme moyen de caractérisation du polymère.

Selon un autre mode de réalisation particulier, la présente invention concerne un polymère à empreintes moléculaires tel que défini ci-dessus, ledit polymère à empreintes moléculaires étant sous la forme d'une poudre ayant une granulométrie comprise de 25 à 40 µm.

Par « 25 à *40 µm* » on entend également les gammes suivantes : de 25 à 35 µm, de 25 à 30 µm, de 30 à 40 µm, de 35 à 40 µm, de 30 à 35 µm, notamment de 25 à 36 µm.

Les particules de la granulométrie telle que définie ci-dessus peuvent être isolées par des procédés connus de l'homme de l'art, comme le tamisage et la sédimentation.

Des particules trop fines, ayant une granulométrie trop basse, pourrait passer au travers d'un fritté positionné dans la partie inférieure d'une cartouche comprenant le MIP. Par ailleurs, des particules trop fines rendent difficile le passage des différents solvants au travers d'une cartouche comprenant le MIP, avec un risque de colmatage de la cartouche.

Des particules trop grosses, ayant une granulométrie trop élevée, ont une moindre accessibilité des sites d'empreinte, par rapport aux particules plus fines.

**Un deuxième objet** de la présente invention est un procédé de préparation d'un polymère à empreintes moléculaires, dans lequel le procédé comprend :
- une étape A de polymérisation d'un monomère et d'un agent de réticulation,
   ladite étape A de polymérisation étant effectuée en présence d'un porogène, d'un initiateur de polymérisation radicalaire, et du chlordécol,

dans lequel le monomère est choisi parmi le 4-vinylpyridine et le 2-vinylpyridine,
dans lequel l'agent de réticulation est choisi parmi l'éthylène glycol diméthacrylate (EGDMA), le triméthylolpropane triméthacrylate (TRIM) et le pentaérythritol triacrylate (PETRA), et dans lequel le porogène est de l'acétonitrile ou du dichlorométhane,
pour obtenir un polymère à empreintes moléculaires contenant du chlordécol.

L'invention concerne un procédé de préparation d'un polymère à empreintes moléculaires, dans lequel le procédé comprend :
- une étape A de polymérisation d'un monomère et d'un agent de réticulation, ladite étape A de polymérisation étant effectuée en présence d'un porogène, d'un initiateur de polymérisation radicalaire, et du chlordécol,

dans lequel le monomère est choisi parmi le 4-vinylpyridine et le 2-vinylpyridine,
dans lequel l'agent de réticulation est choisi parmi l'éthylène glycol diméthacrylate (EGDMA), le triméthylolpropane triméthacrylate (TRIM) et le pentaérythritol triacrylate (PETRA),
dans lequel le porogène est de l'acétonitrile ou du dichlorométhane, et
dans lequel l'initiateur de polymérisation radicalaire est notamment de l'AIBN,
pour obtenir un polymère à empreintes moléculaires contenant du chlordécol.

Les initiateurs de polymérisation radicalaire utilisables dans la présente invention sont tout initiateur connu pour initier une polymérisation radicalaire, dont par exemple : le 2,2,6,6-Tetramethyl-1-piperidinyloxy (TEMPO), l'azobisisobutyronitrile (AIBN), le peroxyde de benzoyle (BPO), le peroxyde de di-tert-butyle (TBP), le peroxyde de tert-butyle (TBHP).

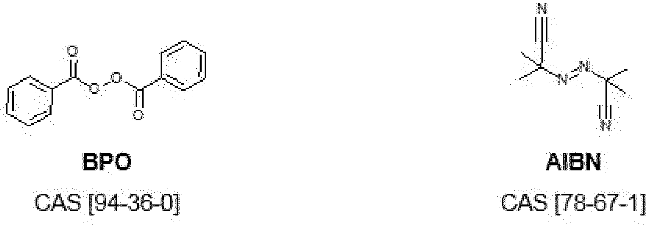

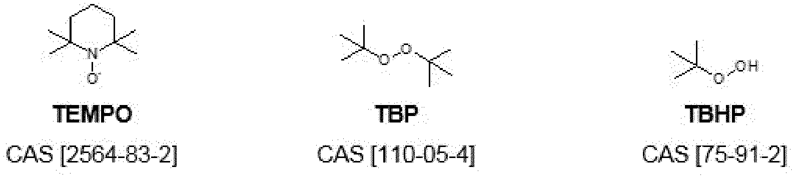

Le porogène, ou solvant porogène, l'acétonitrile ou le dichlorométhane dans la présente invention, permet d'obtenir un polymère de structure poreuse. Les sites d'empreinte sont ainsi accessibles depuis l'extérieur du polymère par les pores formés par ledit porogène. Le porogène assure donc l'accès du chlordécone et/ou du chlordécol aux sites d'empreinte.

La préparation des polymères à empreintes moléculaires selon la présente invention peut être effectuée dans du matériel de laboratoire ou de production classique. Puis, le produit obtenu peut être séché pour éliminer, ou substantiellement éliminer, le solvant porogène. L'impression moléculaire, pour obtenir un polymère à empreintes moléculaires, repose schématiquement sur les stades représentés dans la **Figure 1****.**

Des monomères, par exemple du 4-vinylpyridine dans le cadre de la présente invention, sont mis en présence de la molécule à imprimer (l'empreinte), par exemple du chlordécol dans la présente invention (**Figure 1A**).

Lesdits monomères interagissent de façon réversible avec l'empreinte pour former des complexes monomères-empreinte (**Figure 1B**).

Lesdits complexes monomères-empreinte subissent ensuite une copolymérisation, lors de laquelle les monomères sont polymérisés en présence d'un agent de réticulation, pour obtenir un réseau polymérique tridimensionnel (**Figure 1C**).

L'empreinte peut ensuite être éliminée, du réseau polymérique, pour obtenir le polymère imprimé proprement dit, dépourvu d'empreinte, et possédant des sites, ou cavités, aptes à recevoir une molécule de structure similaire, ou de la même structure que l'empreinte utilisée (**Figure 1D**). Ainsi, les polymères à empreintes moléculaires selon la présente invention peuvent accueillir à la fois le chlordécol et le chlordécone.

Ainsi, selon un mode de réalisation particulier, la présente invention concerne un procédé de préparation tel que défini ci-dessus, comprenant en outre, après l'étape A de polymérisation, une étape B d'élimination du chlordécol du polymère à empreintes moléculaires, notamment par lavage, pour obtenir ledit polymère dépourvu de chlordécol.

L'étape B d'élimination peut être effectuée par lavage, ou rinçage, du polymère avec un solvant capable de rompre les interactions entre le chlordécol et le polymère, permettant ainsi d'éliminer l'empreinte.

L'élimination du chlordécol peut être mise en évidence par analyse des liquides de lavage, par exemple par LC/MS ou GC/MS, et est considérée terminée quand le chlordécol n'est plus détecté dans lesdits liquides de lavage.

L'étape B d'élimination du chlordécol est notamment effectuée avec un solvant polaire, de préférence protique, comme par exemple le méthanol, l'éthanol ou l'isopropanol.

L'étape B d'élimination est de préférence effectuée sur un polymère sous forme de poudre. En effet, à l'issu de l'étape A, un produit sous la forme d'un « bloc » peut être obtenu, ce qui complique la manipulation du produit et ne permet notamment pas un lavage efficace.

Dans ces conditions, le produit issu du stade A de polymérisation doit être broyé pour assurer la forme « *poudre* » du polymère.

Ainsi, selon un autre mode de réalisation particulier, la présente invention concerne donc un procédé de préparation tel que défini ci-dessus, comprenant, entre l'étape A de polymérisation, et l'étape B d'élimination du chlordécol, une étape C de broyage, pour obtenir un polymère à empreintes moléculaires broyé.

L'étape C de broyage peut être réalisée avec du matériel classique comme par exemple un mortier ou un broyeur de type moulin colloïdal.

Selon un mode de réalisation particulier, la présente invention concerne un procédé de préparation tel que défini ci-dessus, comprenant en outre, après l'étape C de broyage, une étape D de tamisage, pour obtenir un polymère à empreintes moléculaires tamisé, ayant notamment une granulométrie comprise de 25 à 40 µm.

L'étape D de tamisage permet d'éliminer les particules ayant une granulométrie supérieure à 40 µm et une partie de celles inferieures à 25 µm.

Les particules supérieures à 40 µm restent dans le tamis de porosité 40 µm, tandis que les particules plus fines, ayant une granulométrie inférieure à 40 µm traversent les mailles du tamis, et passent sur le tamis de porosité 25 µm. Les particules supérieures à 25 µm restent sur le tamis de 25 µm et sont collectées.

Des tamis adaptés au tamisage effectué dans le procédé de la présente invention font parties du matériel classique en laboratoire.

L'étape D de tamisage peut être effectuée avant ou après l'étape B d'élimination du chlordécol, de manière préférée après.

Selon un mode de réalisation particulier, la présente invention concerne un procédé de préparation tel que défini ci-dessus, comprenant en outre, après l'étape D de tamisage, une étape E de sédimentation, pour obtenir un polymère à empreintes moléculaires sédimenté, ayant notamment une granulométrie comprise de 25 à 40 µm.

L'étape E de sédimentation permet d'éliminer les particules « *fines* »*,* i.e. ayant une granulométrie inférieure à 25 µm, si elles n'ont pas été complétement éliminées lors de l'étape D de tamisage. Ces particules fines restent en suspension est peuvent être éliminées par décantation.

L'étape E de sédimentation est effectuée dans un milieu dans lequel le polymère n'est pas soluble, comme par exemple des mélanges aqueux d'alcools tels que le méthanol ou l'éthanol, notamment dans un ratio alcool/eau d'environ 80/20 v/v.

L'étape E de sédimentation peut être effectuée avant ou après l'étape B d'élimination du chlordécol.

Selon un autre mode de réalisation particulier, la présente invention concerne un procédé de préparation tel que défini ci-dessus, comprenant en outre :
après l'étape A de polymérisation, une étape B d'élimination du chlordécol du polymère à empreintes moléculaires, notamment par lavage, pour obtenir ledit polymère dépourvu de chlordécol, ladite étape B d'élimination étant notamment effectuée avec du méthanol,
et optionnellement,
entre l'étape A de polymérisation, et l'étape B d'élimination du chlordécol, une étape C de broyage, pour obtenir un polymère à empreintes moléculaires broyé,
et optionnellement
après l'étape C de broyage, une étape D de tamisage, pour obtenir un polymère à empreintes moléculaires tamisé, ayant notamment une granulométrie comprise de 25 à 40 µm,
et optionnellement
après l'étape D de tamisage, une étape E de sédimentation, pour obtenir un polymère à empreintes moléculaires sédimenté, ayant notamment une granulométrie comprise de 25 à 40 µm, ladite étape E de sédimentation étant notamment effectuée avec un mélange méthanol/eau, en particulier dans un ratio 80/20 v/v.

Selon un autre mode de réalisation particulier, la présente invention concerne un procédé de préparation tel que défini ci-dessus, dans lequel le monomère est le 4-vinylpyridine, et le porogène est de l'acétonitrile.

Selon un autre mode de réalisation particulier, la présente invention concerne un procédé de préparation tel que défini ci-dessus, dans lequel le monomère est le 2-vinylpyridine, et le porogène est du dichlorométhane.

Selon un autre mode de réalisation particulier, la présente invention concerne un procédé de préparation tel que défini ci-dessus, dans lequel l'initiateur de polymérisation utilisé dans l'étape A de polymérisation est du AIBN.

Selon un autre mode de réalisation particulier, la présente invention concerne un procédé de préparation tel que défini ci-dessus, dans lequel l'agent de réticulation utilisé dans l'étape A de polymérisation est l'EGDMA.

Selon un autre mode de réalisation particulier, la présente invention concerne un procédé de préparation tel que défini ci-dessus, dans lequel le ratio molaire monomère : agent de réticulation utilisé dans l'étape A de polymérisation est compris de 3 : 30, de préférence d'environ 4 : 20.

Un ratio monomère : agent de réticulation trop bas conduit à une baisse de la capacité de rétention du polymère, tandis qu'un ratio trop élevé conduit à une perte de sélectivité de rétention.

Selon un autre mode de réalisation particulier, la présente invention concerne un procédé de préparation tel que défini ci-dessus, dans lequel le ratio molaire chlordécol : monomère utilisé dans l'étape A de polymérisation est compris de 0,75 : 6, de préférence d'environ 1 : 4.

Un ratio chlordécol : monomère trop bas conduit à une baisse de la capacité de rétention du polymère, du fait de la formation de moins de sites d'empreintes, tandis qu'un ratio trop élevé conduit à une perte de sélectivité.

Selon un autre mode de réalisation particulier, la présente invention concerne un procédé de préparation tel que défini ci-dessus, dans lequel le ratio molaire chlordécol : agent de réticulation utilisé dans l'étape A de polymérisation est compris de 0,5 : 30, de préférence d'environ 1 : 20.

Un ratio chlordécol : agent de réticulation trop bas conduit à une baisse de la capacité de rétention du polymère, du fait de la formation de moins de sites d'empreintes, tandis qu'un ratio trop élevé conduit à une perte de sélectivité.

Selon un autre mode de réalisation particulier, la présente invention concerne un procédé de préparation tel que défini ci-dessus, dans lequel le ratio chlordécol : monomère : agent de réticulation utilisé dans l'étape A de polymérisation est d'environ 1 : 4 : 20.

Les Inventeurs ont identifié ce ratio comme étant le plus avantageux en termes de capacité de rétention et de sélectivité.

Selon un autre mode de réalisation particulier, la présente invention concerne un procédé de préparation tel que défini ci-dessus, dans lequel l'initiateur de polymérisation est utilisé à raison de 1% molaire par rapport au monomère.

Selon un autre mode de réalisation particulier, la présente invention concerne un procédé de préparation tel que défini ci-dessus, dans lequel :
- le ratio molaire monomère : agent de réticulation utilisé dans l'étape A de polymérisation est compris de 3 : 30, de préférence d'environ 4 : 20, et/ou
- le ratio molaire chlordécol : monomère utilisé dans l'étape A de polymérisation est compris de 0,75 : 6, de préférence d'environ 1 : 4, et/ou
- le ratio molaire chlordécol : agent de réticulation utilisé dans l'étape A de polymérisation est compris de 0,5 : 30, de préférence d'environ 1 : 20,
notamment, dans lequel le ratio chlordécol : monomère : agent de réticulation utilisé dans l'étape A de polymérisation est d'environ 1 : 4 : 20.

Selon un autre mode de réalisation particulier, la présente invention concerne un procédé de préparation tel que défini ci-dessus, dans lequel l'étape A de polymérisation est effectuée soit à une température comprise de 50 à 54 °C, notamment à une température de 54 °C, le porogène étant de l'acétonitrile,
soit à une température comprise de 25 à 30 °C, le porogène étant du dichlorométhane,

A une température trop basse, la faible cinétique de la réaction n'est pas compatible avec un procédé industriellement fiable. Une température trop élevée conduit à une évaporation, du moins partielle, du solvant porogène lors de la synthèse, entrainant des problèmes de solubilité et/ou des polymères non-homogènes.

Selon un autre mode de réalisation particulier, la présente invention concerne un procédé de préparation tel que défini ci-dessus, dans lequel l'étape B d'élimination du chlordécol est effectuée avec du méthanol.

Selon un autre mode de réalisation particulier, la présente invention concerne un procédé de préparation tel que défini ci-dessus, dans lequel l'étape E de sédimentation est effectuée avec un mélange méthanol/eau, notamment dans un ratio 80/20.

Selon un autre mode de réalisation particulier, la présente invention concerne un procédé de préparation tel que défini ci-dessus, comprenant :
- une étape A de polymérisation du 4-vinylpyridine et l'éthylène glycol diméthacrylate, dans de l'acétonitrile, en présence d'AIBN et de chlordécol, pour obtenir un polymère à empreintes moléculaires contenant du chlordécol,
- une étape C de broyage, pour obtenir un polymère à empreintes moléculaires broyé,
- une étape D de tamisage, pour obtenir un polymère à empreintes moléculaires tamisé, ayant notamment une granulométrie inférieure à 40 µm,
- une étape E de sédimentation, pour obtenir un polymère à empreintes moléculaires sédimenté, ayant notamment une granulométrie comprise de 25 à 40 µm, et
- optionnellement une étape B d'élimination du chlordécol du polymère à empreintes moléculaires, notamment par du méthanol, pour obtenir ledit polymère à empreintes moléculaires dépourvu de chlordécol.

L'étape B d'élimination du chlordécol, optionnelle, peut être effectuée avant ou après l'étape D de tamisage ou l'étape E de sédimentation.

**Un troisième objet** de la présente invention est un polymère à empreintes moléculaires susceptible d'être obtenu par le procédé tel que défini ci-dessus.

**Un quatrième objet** de la présente invention est l'utilisation d'un polymère imprimé tel que défini ci-dessus, pour la purification d'un échantillon comprenant le chlordécol et/ou du chlordécone.

Les échantillons pouvant être purifiés dans le cadre de la présente invention peuvent être à la fois des échantillons solides une fois mis en solution, ou des échantillons liquides.

Parmi les échantillons solides on peut notamment citer:
Des échantillons de sol, comme par exemple la terre ou le sable.

Des échantillons alimentaires solides, comme par exemple la viande, le poisson, ou les fruits et légumes.

Des échantillons biologiques solides, comme par exemple les tissus, ou les cheveux.

Parmi les échantillons liquides on peut notamment citer :
Des échantillons biologiques liquides, comme par exemple le sang, le sérum, ou l'urine.

Des échantillons alimentaires liquides, comme par exemple le lait, ou les jus de fruits.

Des échantillons aqueux divers, comme par exemple l'eau de mer, l'eau des rivières, l'eau de pluie, l'eau des lacs, l'eau souterraine (de la nappe phréatique par exemple).

Selon un mode de réalisation particulier, la présente invention concerne l'utilisation telle que définie ci-dessus, dans laquelle l'échantillon est un échantillon de sol, un échantillon aqueux, un échantillon alimentaire ou un échantillon biologique.

**Un quatrième objet** de la présente invention est une cartouche comprenant un polymère à empreintes moléculaires tel que définie ci-dessus.

La cartouche peut avoir une forme comme visualisée dans la **Figure 2** (modifiée de *https:*//*fr.gilson.com*/*FRFR*/*learninghub*/*post*/*the-ins-and-outs-of-a-solid-phase-extraction-spe-workflow.html*), ladite cartouche étant équipée, dans sa partie inférieure, d'un fritté permettant d'éviter que le polymère sorte de la cartouche avec le flux de liquide.

Ledit fritté ayant une porosité compatible avec la granulométrie du polymère à empreintes moléculaires y contenu, à savoir une porosité inférieure à ladite granulométrie.

Un deuxième fritté est posé sur la partie supérieure de la cartouche, afin de permettre de charger la cartouche avec les liquides, sans endommager la surface du polymère contenu dans la cartouche, mais aussi « *bloquer* » le polymère dans la cartouche.

La cartouche peut contenir un polymère à empreintes moléculaires dépourvu d'empreinte (le chlordécol), ou alors elle peut contenir un polymère à empreintes moléculaires avec l'empreinte toujours en place dans les sites d'empreinte.

Dans ce dernier cas la cartouche a été chargée d'un polymère à empreintes moléculaires n'ayant pas subi l'étape B d'élimination du chlordécol telle que définie précédemment, et l'empreinte sera préalablement à éliminer, avant utilisation dans un procédé de purification tel que défini ci-après.

Dans ce qui suit, on entend par « procédé de purification » un procédé permettant d'enrichir un échantillon en chlordécone et/ou en chlordécol, par rapport aux autres composants, ou interférents, pouvant être présent dans l'échantillon à purifier.

**Un cinquième objet** de la présente invention est un procédé de purification d'un échantillon comprenant du chlordécone et/ou du chlordécol, lequel procédé comprenant :
- une étape 1 de percolation dudit échantillon sur un polymère à empreintes moléculaires tel que défini ci-dessus, pour obtenir un polymère à empreintes moléculaires sur lequel le chlordécone et/ou le chlordécol est retenu.

L'invention concerne un procédé de purification d'un échantillon comprenant du chlordécone et/ou du chlordécol, lequel procédé comprenant :
- une étape 1 de percolation dudit échantillon sur un polymère à empreintes moléculaires tel que défini ci-dessus, en particulier le 4-VP MIP, pour obtenir un polymère à empreintes moléculaires sur lequel le chlordécone et/ou le chlordécol est retenu,

dans lequel l'échantillon à percoler est notamment un échantillon aqueux, un échantillon alimentaire, un échantillon de sol ou un échantillon biologique,
ladite étape 1 de percolation est notamment effectuée avec de l'acétonitrile ou de l'hexane ou de l'heptane.

Dans un mode de réalisation particulier, l'invention concerne un procédé de purification d'un échantillon comprenant du chlordécone et/ou du chlordécol, lequel procédé comprenant :
- une étape 1 de percolation dudit échantillon sur un polymère à empreintes moléculaires tel que défini ci-dessus, en particulier le 4-VP MIP, pour obtenir un polymère à empreintes moléculaires sur lequel le chlordécone et/ou le chlordécol est retenu,
dans lequel l'échantillon à percoler est notamment un extrait organique d'un échantillon aqueux, d'un échantillon alimentaire, d'un échantillon de sol ou d'un échantillon biologique, ladite étape 1 de percolation est notamment effectuée avec de l'acétonitrile, de l'hexane ou de l'heptane et le dichlorométhane.

La percolation s'effectue notamment sur une cartouche telle que définie ci-dessus, contenant un polymère à empreintes moléculaires, par percolation d'une solution comprenant le chlordécone et/ou le chlordécol à travers d'un polymère à empreintes moléculaires contenu dans ladite cartouche, comme schématisé dans la **Figure 2****.** Le chlordécone et/ou le chlordécol est retenu par le polymère grâce aux sites d'empreintes.

Le liquide récupéré en bas de la cartouche, substantiellement dépourvu de chlordécone et/ou de chlordécol est appelé, dans ce qui suit, la, ou les « *fraction(s) de percolation ».* De la même façon, le liquide récupéré lors de l'étape de lavage est appelé la, ou les *« fraction(s) de lavage »,* ou alors liquides de lavage, et le liquide récupéré lors de l'étape d'élution est appelé la, ou les *« fraction(s) d'élution »,* ou éluât.

Le solvant de percolation, c'est-à-dire le solvant dans lequel l'échantillon à purifier a été préalablement solubilisé, ou dilué, est un solvant qui n'élue pas le chlordécone et/ou le chlordécol, notamment un solvant non-protique, en particulier choisi parmi l'acétonitrile, l'hexane, l'heptane et le dichlorométhane.

Le solvant de percolation peut être ajouté de différentes façons, en fonction de l'échantillon à purifier.

Par exemple, un échantillon de sol solide, doit être préparé et mis en solution dans le solvant de percolation, afin d'y extraire le chlordécone et/ou le chlordécol.

A contrario, un échantillon liquide, comme par exemple du sérum, peut par exemple être dilué par le solvant de percolation, puis le solvant de percolation comprenant le chlordécone et/ou le chlordécol peut être séparé par décantation, éventuellement, dans le cas d'acétonitrile par exemple, après saturation de la phase aqueuse par ajout de sels, tels que le chlorure de sodium.

Dans le cas où l'ajout du solvant de percolation à l'échantillon à purifier conduit à la formation d'un précipité, une étape de centrifugation et/ou filtration peut être mise en œuvre avant la percolation sur le polymère. Par exemple, dans le cas d'un sérum, il est possible que des protéines, non-solubles dans un solvant comme de l'acétonitrile, précipitent.

L'échantillon à purifier peut également subir d'autres pré-traitements connus de l'homme de l'art, comme par exemple par la méthode QuEChERS pour les échantillons de type denrée alimentaire.

Selon un mode de réalisation particulier, la présente invention concerne un procédé de purification tel que défini ci-dessus, comprenant en outre, avant l'étape 1 de percolation :
- une étape 0 de conditionnement du polymère à empreintes moléculaires, pour obtenir un polymère à empreintes moléculaires conditionné.

Dans ce mode de réalisation, le polymère est mis en état d'utilisation.

Selon un mode de réalisation particulier, l'étape 0 de conditionnement est réalisée avec le solvant utilisé lors de l'étape 1 de percolation. Il s'agit de conditionner la cartouche avec le solvant utilisé lors de l'étape 1 de percolation.

Selon un mode de réalisation particulier, l'étape 0 de conditionnement est réalisée avec de l'hexane et l'étape 1 de percolation est réalisée avec l'heptane.

Selon un mode de réalisation particulier, l'étape 0 de conditionnement est réalisée avec de l'heptane et l'étape 1 de percolation est réalisée avec l'hexane.

Avantageusement, l'étape 0 de conditionnement peut être réalisée avec un solvant choisi parmi l'acétonitrile, l'hexane, l'heptane et le dichlorométhane.

L'étape de conditionnement permet de mettre à l'équilibre le polymère avec le solvant afin de permettre la rétention ultérieure de la chlordécone et/ou du chlordécol, et de préparer les sites d'empreinte pour favoriser la rétention du chlordécone et/ou du chlordécol.

Selon un autre mode de réalisation particulier, la présente invention concerne un procédé de purification tel que défini ci-dessus, comprenant en outre, après l'étape 1 de percolation :
- une étape 2 de lavage du polymère à empreintes moléculaires sur lequel le chlordécone et/ou le chlordécol est percolé, pour obtenir un polymère à empreintes moléculaires lavé.

L'étape 2 de lavage permet d'éliminer, totalement ou partiellement, les composants autres que le chlordécone et/ou le chlordécol. L'étape 2 de lavage peut être réalisée par un seul lavage avec un solvant ou par une succession, d'au moins deux lavages, avec des solvants identiques ou différents.

Selon un autre mode de réalisation particulier, la présente invention concerne un procédé de purification tel que défini ci-dessus, dans lequel l'étape 2 de lavage est effectuée avec un seul lavage avec un solvant.

Selon un autre mode de réalisation particulier, la présente invention concerne un procédé de purification tel que défini ci-dessus, dans lequel l'étape 2 de lavage est effectuée avec une succession, d'au moins deux lavages, avec des solvants identiques ou différents.

Lorsque des solvants différents sont utilisés, une étape de séchage peut être intercalée entre les deux étapes de lavages avec des solvants différents. L'étape de séchage permet ainsi d'éviter la possible formation d'une émulsion dans la cartouche entre des solvants non miscibles tels que l'heptane et l' acétonitrile/ le méthanol.

Avantageusement l'étape de séchage est réalisée sous vide ou sous air. De préférence l'étape de séchage est réalisée sous air, par exemple par un passage d'air sur la cartouche. Le séchage sous air a pour avantage d'être moins chronophage et présente une plus grande facilité de mise en œuvre.

Selon un autre mode de réalisation particulier, la présente invention concerne un procédé de purification tel que défini ci-dessus, dans lequel l'étape 2 de lavage comprend au moins une étape de séchage, de préférence sous air ou sous azote.

Selon un autre mode de réalisation particulier, la présente invention concerne un procédé de purification tel que défini ci-dessus, comprenant en outre, après l'étape 2 de lavage :
- une étape 3 d'élution du chlordécone et/ou du chlordécol, pour obtenir un éluât comprenant le chlordécone et/ou le chlordécol.

Ainsi, un éluât est obtenu qui est purifié par rapport à l'échantillon à purifier, déposé lors de l'étape 1 de percolation. Au moins une partie des impuretés ont été éliminées lors de l'étape 2 de lavage.

L'éluât obtenu contient un ratio « (chlordécone et/ou chlordécol) / impuretés », plus élevé par rapport à l'échantillon à purifier.

L'étape 3 d'élution est notamment effectuée avec un solvant polaire, de préférence protique, comme par exemple le méthanol, l'éthanol ou l'isopropanol.

Selon un autre mode de réalisation particulier, la présente invention concerne un procédé de purification tel que défini ci-dessus, comprenant en outre, après l'étape 3 d'élution :
- une étape 4 détection du chlordécone et/ou du chlordécol par analyse de l'éluât, notamment par LC/MS ou par GC/MS.

L'étape 4 permet de détecter la présence de chlordécone et/ou de chlordécol dans l'éluât issu de l'étape 3 d'élution.

Une quantification du chlordécone et/ou du chlordécol est également possible par traitement du signal de détection par des méthodes connues de l'homme de l'art.

Ainsi, Selon un autre mode de réalisation particulier, la présente invention concerne un procédé de purification tel que défini ci-dessus, comprenant en outre, après l'étape 3 d'élution :
- une étape 4 de quantification du chlordécone et/ou du chlordécol par analyse de l'éluât, notamment par LC/MS ou par GC/MS.

Selon un autre mode de réalisation particulier, la présente invention concerne un procédé de purification tel que défini ci-dessus, dans lequel l'étape 0 de conditionnement est effectuée avec de l'acétonitrile, de l'hexane ou de l'heptane.

Selon un autre mode de réalisation particulier, la présente invention concerne un procédé de purification tel que défini ci-dessus, dans lequel l'étape 1 de percolation est effectuée avec de l'acétonitrile, de l'hexane ou de l'heptane.

Selon un autre mode de réalisation particulier, la présente invention concerne un procédé de purification tel que défini ci-dessus, dans lequel l'étape 2 de lavage est effectuée avec un mélange acétonitrile/méthanol, notamment dans un ratio 95/5.

Selon un autre mode de réalisation particulier, la présente invention concerne un procédé de purification tel que défini ci-dessus, dans lequel l'étape 2 de lavage est effectuée avec un premier lavage avec l'heptane suivi d'une étape de séchage et un deuxième lavage avec un mélange acétonitrile/méthanol, notamment dans un ratio 95/5.

Selon un autre mode de réalisation particulier, la présente invention concerne un procédé de purification tel que défini ci-dessus, dans lequel l'étape 3 d'élution est effectuée avec du méthanol.

Selon un autre mode de réalisation particulier, la présente invention concerne un procédé de purification tel que défini ci-dessus, dans lequel l'échantillon à percoler lors de l'étape 1 est un échantillon aqueux, un échantillon alimentaire, un échantillon de sol, ou un échantillon biologique.

Selon un autre mode de réalisation particulier, la présente invention concerne un procédé de purification tel que défini ci-dessus, comprenant en outre :
- avant l'étape 1 de percolation, une étape 0 de conditionnement, du polymère à empreintes moléculaires, en particulier le 4-VP MIP, pour obtenir un polymère à empreintes moléculaires conditionné,
   ladite étape 0 de conditionnement étant notamment effectuée avec de l'acétonitrile ou de l'hexane ou de l'heptane, en particulier avec de l'acétonitrile.
   et/ou
- après l'étape 1 de percolation, une étape 2 de lavage du polymère à empreintes moléculaires sur lequel le chlordécone et/ou le chlordécol est percolé, pour obtenir un polymère à empreintes moléculaires lavé,
   ladite étape 2 de lavage étant notamment effectuée avec un mélange acétonitrile/méthanol, notamment dans un ratio 95/5,
   et optionnellement,
- après l'étape 2 de lavage, une étape 3 d'élution du chlordécone, pour obtenir un éluât comprenant le chlordécone et/ou le chlordécol,
   ladite étape 3 d'élution étant notamment effectuée avec du méthanol,
   et optionnellement,
- après l'étape 3 d'élution, une étape 4 détection du chlordécone par analyse de l'éluât, notamment par LC/MS ou GC/MS.

Selon un autre mode de réalisation particulier, la présente invention concerne un procédé de purification tel que défini ci-dessus, comprenant en outre :
- avant l'étape 1 de percolation, une étape 0 de conditionnement, du polymère à empreintes moléculaires, en particulier le 4-VP MIP, pour obtenir un polymère à empreintes moléculaires conditionné,
   ladite étape 0 de conditionnement étant notamment effectuée avec de l'acétonitrile ou de l'hexane ou de l'heptane, en particulier dans l'heptane.
   et/ou
- après l'étape 1 de percolation, une étape 2 de lavage du polymère à empreintes moléculaires sur lequel le chlordécone et/ou le chlordécol est percolé, pour obtenir un polymère à empreintes moléculaires lavé,
   ladite étape 2 de lavage étant notamment effectuée avec un premier lavage avec l'heptane suivi d'une étape de séchage et un deuxième lavage avec un mélange acétonitrile/méthanol, notamment dans un ratio 95/5,
   et optionnellement,
- après l'étape 2 de lavage, une étape 3 d'élution du chlordécone, pour obtenir un éluât comprenant le chlordécone et/ou le chlordécol,
   ladite étape 3 d'élution étant notamment effectuée avec du méthanol,
   et optionnellement,
- après l'étape 3 d'élution, une étape 4 de détection du chlordécone par analyse de l'éluât, notamment par LC/MS ou LC/MS/MS ou GC/MS ou GC/MS/MS.

Selon un autre mode de réalisation particulier, la présente invention concerne un procédé de purification tel que défini ci-dessus, comprenant les étapes suivantes :
- une étape 0 de conditionnement du polymère à empreintes moléculaires, en particulier le 4-VP MIP, notamment par de l'acétonitrile ou de l'hexane ou de l'heptane, pour obtenir un polymère à empreintes moléculaires conditionné,
- une étape 1 de percolation dudit échantillon sur ledit polymère à empreintes moléculaires conditionné, notamment dans de l'acétonitrile ou de l'hexane ou de l'heptane, pour obtenir un polymère à empreintes moléculaires sur lequel le chlordécone et/ou le chlordécol est retenu,
- une étape 2 de lavage du polymère à empreintes moléculaires sur lequel le chlordécone et/ou le chlordécol est retenu, notamment par un mélange d'acétonitrile/méthanol 95/5, pour obtenir un polymère à empreintes moléculaires lavé, et
- une étape 3 d'élution du chlordécone et/ou du chlordécol, notamment avec le méthanol, pour obtenir un éluât comprenant le chlordécone et/ou le chlordécol.

Selon un autre mode de réalisation particulier, la présente invention concerne un procédé de purification tel que défini ci-dessus, comprenant les étapes suivantes :
- une étape 0 de conditionnement du polymère à empreintes moléculaires, en particulier le 4-VP MIP, notamment par de l'acétonitrile, pour obtenir un polymère à empreintes moléculaires conditionné,
- une étape 1 de percolation dudit échantillon sur ledit polymère à empreintes moléculaires conditionné, notamment dans de l'acétonitrile, pour obtenir un polymère à empreintes moléculaires sur lequel le chlordécone et/ou le chlordécol est retenu,
- une étape 2 de lavage du polymère à empreintes moléculaires sur lequel le chlordécone et/ou le chlordécol est retenu, notamment par un mélange d'acétonitrile/méthanol 95/5, pour obtenir un polymère à empreintes moléculaires lavé, et
- une étape 3 d'élution du chlordécone et/ou du chlordécol, notamment avec le méthanol, pour obtenir un éluât comprenant le chlordécone et/ou le chlordécol.

Selon un autre mode de réalisation particulier, la présente invention concerne un procédé de purification tel que défini ci-dessus, comprenant les étapes suivantes :
- une étape 0 de conditionnement du polymère à empreintes moléculaires, en particulier le 4-VP MIP, notamment par de l'acétonitrile ou de l'hexane ou de l'heptane, pour obtenir un polymère à empreintes moléculaires conditionné,
- une étape 1 de percolation dudit échantillon sur ledit polymère à empreintes moléculaires conditionné, notamment dans de l'hexane ou de l'heptane, pour obtenir un polymère à empreintes moléculaires sur lequel le chlordécone et/ou le chlordécol est retenu,
- une étape 2 de lavage du polymère à empreintes moléculaires sur lequel le chlordécone et/ou le chlordécol est retenu, notamment par un premier lavage avec l'heptane ou hexane suivi d'une étape de séchage et un deuxième lavage avec un mélange acétonitrile/méthanol, notamment dans un ratio 95/5, pour obtenir un polymère à empreintes moléculaires lavé, et
- une étape 3 d'élution du chlordécone et/ou du chlordécol, notamment avec le méthanol, pour obtenir un éluât comprenant le chlordécone et/ou le chlordécol.

Selon un autre mode de réalisation particulier, la présente invention concerne un procédé de purification tel que défini ci-dessus, comprenant les étapes suivantes :
- une étape 0 de conditionnement du polymère à empreintes moléculaires, en particulier le 4-VP MIP, notamment par de l'hexane ou de l'heptane, en particulier l'heptane, pour obtenir un polymère à empreintes moléculaires conditionné,
- une étape 1 de percolation dudit échantillon sur ledit polymère à empreintes moléculaires conditionné, notamment dans de l'acétonitrile, pour obtenir un polymère à empreintes moléculaires sur lequel le chlordécone et/ou le chlordécol est retenu,
- une étape 2 de lavage du polymère à empreintes moléculaires sur lequel le chlordécone et/ou le chlordécol est retenu, notamment par un premier lavage avec l'heptane suivi d'une étape de séchage et un deuxième lavage avec un mélange acétonitrile/méthanol, notamment dans un ratio 95/5, pour obtenir un polymère à empreintes moléculaires lavé, et
- une étape 3 d'élution du chlordécone et/ou du chlordécol, notamment avec le méthanol, pour obtenir un éluât comprenant le chlordécone et/ou le chlordécol.

Selon un autre mode de réalisation particulier, la présente invention concerne un procédé de purification tel que défini ci-dessus, comprenant les étapes suivantes :
- une étape 0 de conditionnement du polymère à empreintes moléculaires, en particulier le 4-VP MIP, notamment par de l'hexane ou de l'heptane, en particulier l'heptane, pour obtenir un polymère à empreintes moléculaires conditionné,
- une étape 1 de percolation dudit échantillon sur ledit polymère à empreintes moléculaires conditionné, notamment dans de l'hexane ou de l'heptane, en particulier dans l'heptane, pour obtenir un polymère à empreintes moléculaires sur lequel le chlordécone et/ou le chlordécol est retenu,
- une étape 2 de lavage du polymère à empreintes moléculaires sur lequel le chlordécone et/ou le chlordécol est retenu, notamment par un premier lavage avec l'heptane suivi d'une étape de séchage et un deuxième lavage avec un mélange acétonitrile/méthanol, notamment dans un ratio 95/5, pour obtenir un polymère à empreintes moléculaires lavé, et
- une étape 3 d'élution du chlordécone et/ou du chlordécol, notamment avec le méthanol, pour obtenir un éluât comprenant le chlordécone et/ou le chlordécol.

Les exemples et figures suivants illustrent l'invention, sans en limiter la portée.
La **Figure 1** représente le principe général de polymères à empreintes moléculaires. **A** représente les différents réactifs ; les monomères **1** l'agent réticulant **2,** et l'empreinte **3. B** représente les interactions entre les monomères et l'empreinte, avant polymérisation. **C** représente la polymérisation des monomères autour de l'empreinte et D représente la libération des cavités par lavage de l'empreinte.
La **Figure 2** représente le principe général d'utilisation d'un MIP dans la purification d'un échantillon. **0** représente l'étape 0 de conditionnement, **1** représente l'étape 1 de percolation, **2** représente l'étape 2 de lavage, et **3** représente l'étape 3 d'élution,
   • représente les analytes, le chlordécone et/ou le chlordécol dans la présente invention,
   ▲ ●■ représentent les interférents, à savoir les composés autres que le chlordécone et/ou le chlordécol dans la présente invention.

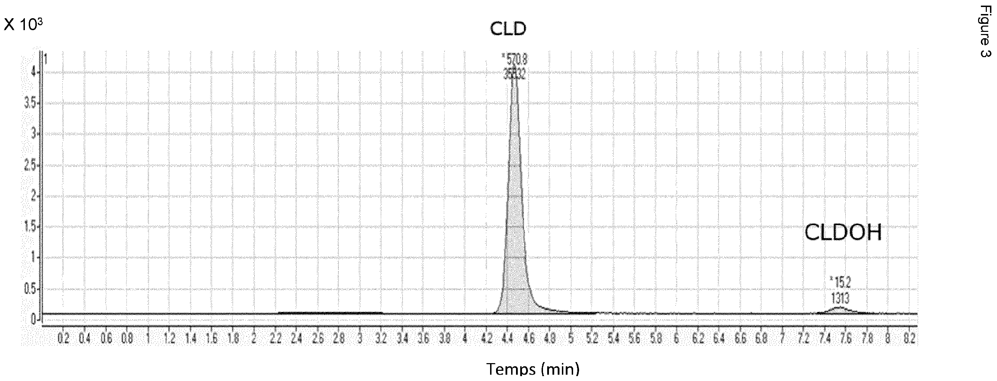

La **Figure 3** représente le profil LC/MS obtenu avec la méthode analytique de l'exemple A.

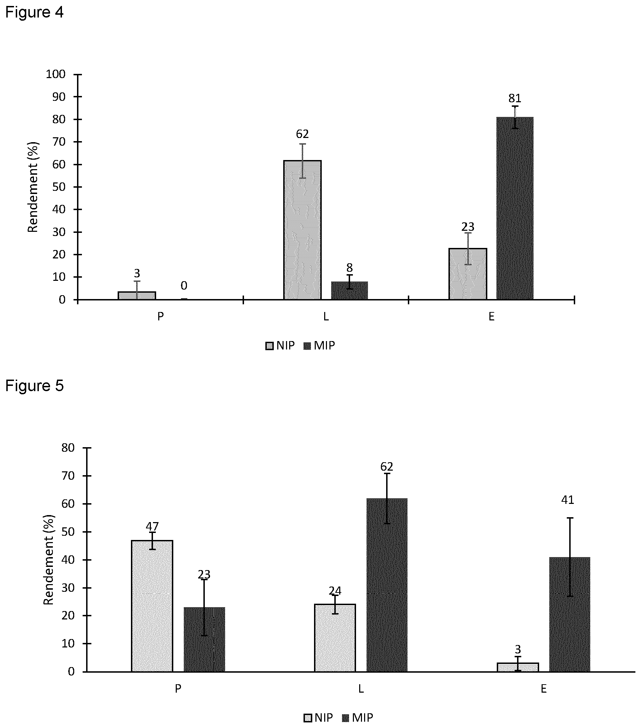

La **Figure 4** représente le rendement d'extraction pour le **chlordécone** présent dans les différentes fractions obtenues dans l'exemple 3. Les barres foncées représentent la quantité de chlordécone en utilisant les MIP, et les barres claires représentent la quantité de chlordécone en utilisant les NIP. **P** représente les fractions de percolation, **L** représente les fractions de lavage, et **E** représente les fractions d'élution.
La **Figure 5** représente le rendement d'extraction pour le **chlordécol** présent dans les différentes fractions obtenues dans l'exemple 3. Les barres foncées représentent la quantité de chlordécol en utilisant les MIP, et les barres claires représentent la quantité de chlordécol en utilisant les NIP. **P** représente les fractions de percolation, **L** représente les fractions de lavage, et **E** représente les fractions d'élution.

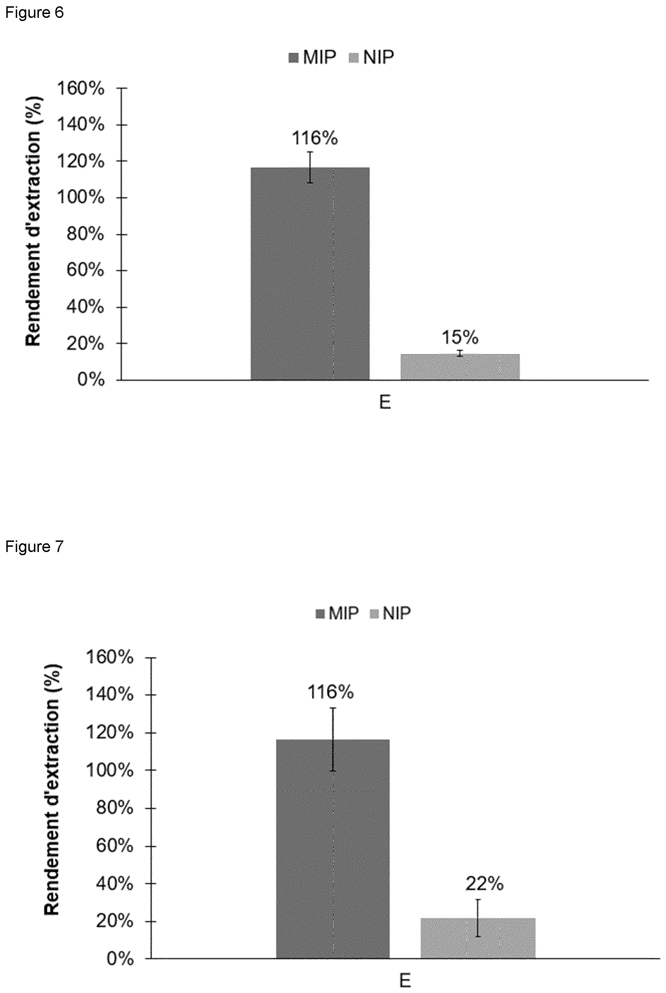

La **Figure 6** représente le rendement d'extraction pour le **chlordécone** présent dans la fraction d'élution (E) obtenue dans l'exemple 6 d'un extrait de **saumon** dans **l'hexane.** La barre foncée représente le rendement de chlordécone en utilisant les MIP, et la barre claire représentent le rendement de chlordécone en utilisant les NIP.
La **Figure 7** représente le rendement d'extraction pour le **chlordécone** présent dans la fraction d'élution (E) obtenue dans l'exemple 6 d'un extrait de **tomate** dans l'**hexane**. La barre foncée représente le rendement de chlordécone en utilisant les MIP, et la barre claire représentent le rendement de chlordécone en utilisant les NIP.

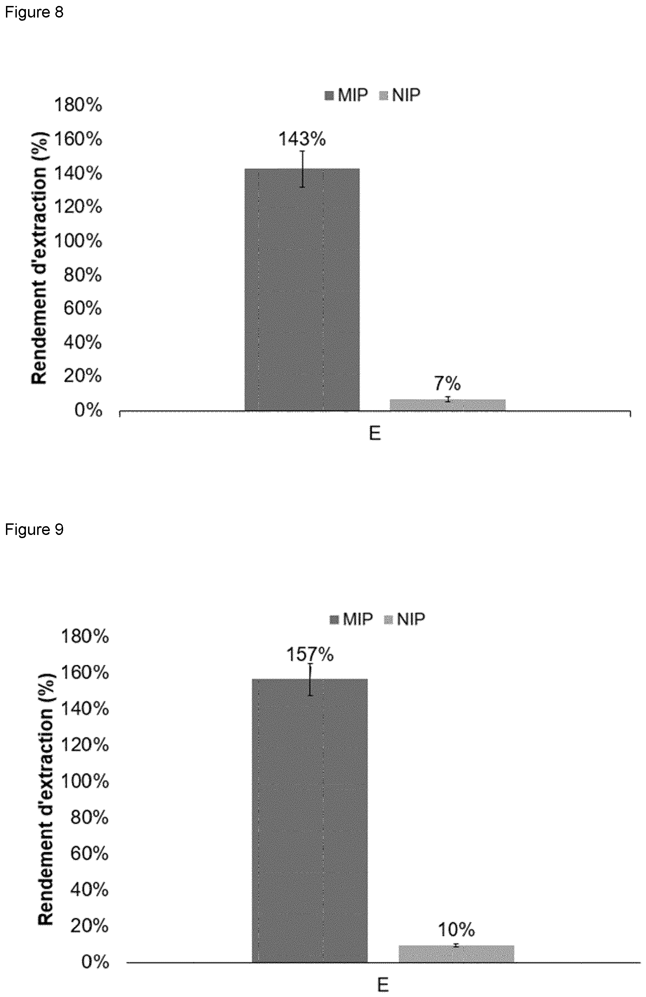

La **Figure 8** représente le rendement de **chlordécone** présent dans la fraction d'élution (E) obtenue dans l'exemple 6 d'un extrait de **saumon** dans l'**acétonitrile.** La barre foncée représente le rendement de chlordécone en utilisant les MIP, et la barre claire représentent le rendement de chlordécone en utilisant les NIP.
La **Figure 9** représente le rendement de **chlordécone** présent dans la fraction d'élution (E) obtenue dans l'exemple 6 d'un extrait de **tomate** dans l'**acétonitrile.** La barre foncée représente le rendement de chlordécone en utilisant les MIP, et la barre claire représentent le rendement de chlordécone en utilisant les NIP.

### EXEMPLES

### Exemple A - Méthode analytique de détection

La détection et la quantification du chlordécone et du chlordécol dans les échantillons ont été effectuées par LC/MS, dans les conditions du tableau 1.

**Tableau 1 conditions LC/MS**

| | | |
|---|---|---|
| *Conditions chromatographiques* | Colonne | Atlantis C18 5 µm, 2,1 x 150 mm |
| | Température de la colonne | 40°C |
| | Phase Mobile | A/ Eau + 0,1% Acide Formique (AF) |
| | | B/ ACN + 0,1% AF |
| | | Mode isocratique : 40% de A /60% de B |
| | Débit | 300 µL/min |
| | Volume d'injection | 5 µL |
| *Paramètres du spectromètre de masse* | Appareil | Triple quadripôle (Agilent) |
| | Source | ESI en mode négatif |
| | Température | 340°C |
| | Débit du gaz | 10 l/min |
| | Nébuliseur | 45 psi |
| | Tension du capillaire | 3000 V |
| | Tension de la cellule d'accélération | 3 V |

### Exemple 1 - Préparation d'un polymère à empreintes moléculaires (MIP)

Dans un flacon en verre, du chlordécol (1 mmol) et du 4-vinylpyridine (4 mmol) ont été mélangés dans de l'acétonitrile (1,8 ml) et le mélange a été placé dans un bain aux ultrasons. Après 10 minutes de sonication, de l'éthylène glycol diméthacrylate (20 mmol) a été ajouté, puis le mélange a été purgé pendant 10 minutes avec un flux de N₂ pour éliminer l'oxygène dissous. De l'AIBN (0,2 mmol), a été ajouté au mélange réactionnel et le flacon de verre a été fermé hermétiquement et placé dans un bain-marie à 60°C pendant 24 heures. Les flacons ont ensuite été brisés, et le polymère obtenu a été broyé et tamisé et les particules de tailles comprises entre 25 et 36 µm ont été collectées.

Une étape de sédimentation avec 4 x 5 mL d'un mélange méthanol/eau 80/20 v/v, a été réalisée pour éliminer les particules les plus fines, et les particules ayant une granulométrie comprise de de 25 à 40 µm ont été mises en cartouche entre 2 frittés pour pouvoir être utilisées comme support d'extraction sur phase solide. Ainsi, des cartouches comprenant 35 mg de MIP ont été préparées.

Le MIP ainsi contenu dans les cartouches contient encore l'empreinte, à savoir le chlordécol, qui a été éliminé par passage de méthanol, préalablement à l'utilisation dans les exemples ci-après.

### Exemple 2 - Préparation d'un polymère dépourvu d'empreintes moléculaires (NIP)

Un support non imprimé (NIP) a été synthétisé dans les mêmes conditions qu'utilisées dans l'exemple 1, mais sans introduire le chlordécol dans le mélange réactionnel.

### Exemple 3 - Caractérisation du MIP

Le MIP (35 mg), contenu dans une cartouche, obtenu selon l'exemple 1 a été conditionné par passage d'1 ml d'acétonitrile (étape 0 de conditionnement). Ensuite, une solution d'1 mL de chlordécone (CLD) à 0.2 µg/mL et de chlordécol (CLDOH) à 0,2 µg/mL dans de l'acétonitrile a été percolé sur le MIP, par passage de ladite solution sur la cartouche (étape 1 de percolation).

Le MIP a ensuite été lavé avec 1.5 mL d'un mélange acétonitrile/méthanol, 95/5, v/v (étape 2 de lavage). Enfin, le chlordécone et le chlordécol ont été élué par passage de 3 mL de méthanol (étape 3 d'élution).

La même procédure a été mise en oeuvre sur un NIP, obtenu selon l'exemple 2.

Les expériences sur le MIP, et sur le NIP, ont été réalisées 3 fois sur 3 cartouches issues de 3 lots de MIP et de NIP différents.

Pour chacune des expériences, les fractions de conditionnement, de percolation, de lavage, et d'élution, récupérées en bas des cartouches, ont été analysées par la procédure LC/MS de l'exemple A. Ainsi, la quantité de chlordécone et de chlordécol, présent dans les différentes fractions a été déterminée et la moyenne des résultats a été calculée sur les 3 expériences (MIP et NIP).

La **Figure 4** montre les résultats obtenus pour le chlordécone. L'étape de percolation permet de retenir, sur le MIP, la totalité de chlordécone contenue dans l'échantillon percolé. Lors de l'étape de lavage, seul 8% de chlordécone est lavé de la cartouche, dans le cas du MIP, tandis que la majorité de chlordécone est lavée dans le cas du NIP. **Ces résultats montrent que le MIP a la capacité de retenir le chlordécone,** par rapport au NIP qui ne retient pas ce produit. L'étape d'élution permet ensuite de récupérer le chlordécone retenu sur le MIP.

La **Figure 5** montre les résultats obtenus pour le chlordécol, qui est plus retenu sur le MIP que sur le NIP.

### Exemple 4 - Purification/détection d'un échantillon de sérum bovin commercial (sigma Aldrich)

Après prélèvement de l'échantillon, 1.5 mL de sérum ont été traités avec 4,5 ml d'acétonitrile (précipitation des protéines). 4 mL d'eau a été ajoutée au surnageant ainsi obtenu. Des sels (QuEChERS Extraction Packets, composés de 4 g de sulfate de magnésium anhydre, 1 g de chlorure de sodium, 1 g de dihydrate de trisodium citrate, et de 0,5 g sesquihydrate de disodiumdihydrogencitrate), permettant de transférer le chlordécone vers la phase acétonitrile, ont été ajoutés.

Les étapes de conditionnement du MIP, de percolation, de lavage et d'élution ont été effectuées sur une cartouche selon l'exemple 1, et utilisant la procédure de l'exemple 3. Une analyse LC/MS des différentes fractions montre que pour le chlordécone, aucune perte n'est observée lors du conditionnement, une perte de 8% est observée lors du lavage, et que 81% du chlordécone est récupéré lors de l'élution sur le MIP.

Cette analyse montre que pour le chlordécone, 3% de perte est observé lors de la percolation, une perte de 62% est observée lors du lavage, et que seul 23% du chlordécone est récupéré lors de l'élution sur le polymère non imprimé.

Ceci démontre la spécificité du MIP vis-à-vis du chlordécone.

### Exemple 5 - Purification/détection d'un échantillon alimentaire

Pour un échantillon alimentaire, 5g d'échantillon broyé sont prélevés, auxquels sont ajoutés 10 mL d'eau ultrapure et 10 mL d'acétonitrile. Après agitation, et ajout de sels composés de 4 g de sulfate de magnésium anhydre, 1 g de chlorure de sodium, 1 g de dihydrate de trisodium citrate, et de 0,5 g sesquihydrate de disodiumdihydrogencitrate, la phase acétonitrile est percolée sur le support selon l'exemple 1. Une spécificité du MIP vis-à-vis du chlordécone et du chlordécol est observée.

### Exemple 6 - Purification/détection d'un échantillon alimentaire de saumon dans l'hexane

Pour préparer l'extrait de saumon dans l'hexane par Extraction Liquide-Liquide (LLE), le saumon a été broyé à l'aide d'un broyeur-mixeur à couteaux de la marque Grindomix pendant 30 secondes à 7000 tr/min.

Une prise de 5 grammes d'échantillon est pesée dans un tube à centrifuger de 50 mL puis 10 mL d'hexane sont ajoutés et le tube est ensuite vortexé pendant 10 min puis centrifugé pendant 5 min à 4000 tr/min. Le surnageant est prélevé puis filtré pour obtenir l'extrait.

Un dopage de chlordécone (CLD) à 20 µg.kg^{- 1} est effectué dans le filtrat. Le dopage avant la SPE permet d'évaluer l'apport du MIP 4-VP sur l'extraction de la CLD de l'échantillon. La quantité introduite de CLD sert de référence (100%) et l'ensemble du rendement d'extraction expérimental obtenu est calculé à partir de cette quantité.

Le MIP (35 mg), contenu dans une cartouche, obtenu selon l'exemple 1 a été conditionné par passage d'1 mL d'hexane (étape 0 de conditionnement). Ensuite, une solution d'1 mL d'extrait de saumon dans l'hexane dopée avec la chlordécone (CLD) a été percolée sur le MIP, par passage de ladite solution sur la cartouche (étape 1 de percolation).

Le MIP a ensuite été lavé avec 300 µL d'heptane, séché sous air avec environ 20 mL, et ensuite lavé avec 1 mL d'un mélange acétonitrile/méthanol, 95/5, v/v (étape 2 de lavage). Enfin, le chlordécone a été élué par passage de 3 mL de méthanol (étape 3 d'élution).

La même procédure a été mise en oeuvre sur un NIP, obtenu selon l'exemple 2.

La procédure globale, à savoir l'extraction LLE, l'étape 0 de conditionnement, l'étape 1 de percolation, l'étape 2 de lavage, l'étape 3 d'élution et l'étape 4 de détection a été réalisée 3 fois sur le MIP, et sur le NIP. Les expériences ont été réalisées sur 3 cartouches issues de 3 lots de MIP et de NIP différents. Pour éviter tout biais, le même volume de solvant est percolé à travers le support de contrôle (NIP).

Pour chacune des expériences, seule la fraction d'élution (E), récupérée en bas des cartouches, a été analysée par la procédure LC/MS. Ainsi, la quantité de chlordécone présent dans la fraction d'élution a été déterminée et la moyenne des résultats a été calculée sur les 3 expériences (MIP et NIP).

La **Figure 6** montre les résultats obtenus pour le chlordécone dans une matrice alimentaire de **saumon extraite dans l'hexane.**

L'étape de percolation permet de retenir, sur le MIP, la totalité de chlordécone contenue dans l'échantillon percolé. En effet l'étape d'élution permet ensuite de récupérer la totalité du chlordécone retenu sur le MIP (rendement de CLD estimé dans l'éluât de 116 ±8 %). **Ces résultats montrent que le MIP a la capacité de retenir le chlordécone dans un extrait d'échantillon alimentaire de saumon extrait dans l'hexane et de le restituer par élution,** par rapport au NIP qui ne retient pas ce produit (7%). La sélectivité du MIP vis-à-vis du chlordécone est maintenue en milieu réel avec un extrait de saumon dans l'hexane.

### Exemple 7 - Purification/détection d'un échantillon alimentaire de tomate dans l'hexane

Le même protocole que l'exemple 6 a été réalisé avec un échantillon alimentaire une matrice de tomate.

La **Figure 7** montre les résultats obtenus pour le chlordécone dans une matrice alimentaire de **tomate extraite dans l'hexane.** L'étape de percolation permet de retenir, sur le MIP, la totalité de chlordécone contenue dans l'échantillon percolé. En effet l'étape d'élution permet ensuite de récupérer le chlordécone retenu sur le MIP avec un rendement de CLD estimé dans l'éluât de 116 %. **Ces résultats montrent que le MIP a la capacité de retenir le chlordécone dans un extrait d'échantillon alimentaire de tomate et de le restituer par élution,** par rapport au NIP qui ne retient pas ce produit (10%). La sélectivité du MIP vis-à-vis du chlordécone est maintenue en milieu réel avec un extrait de tomate dans l'hexane.

### Exemple 8 - Purification/détection d'un échantillon alimentaire de saumon dans l'acétonitrile

Pour préparer l'extrait de saumon dans l'hexane par Extraction Liquide-Liquide (LLE), le saumon a été broyé à l'aide d'un broyeur-mixeur à couteaux de la marque Grindomix pendant 30 secondes à 7000 tr/min.

Une prise de 5 grammes d'échantillon est pesée dans un tube à centrifuger de 50 mL puis 10 mL d'eau ultrapure et 10 mL d'acétonitrile sont ajoutés et le tube est ensuite vortexé pendant 10 min puis centrifugé pendant 5 min à 4000 tr/min. Le surnageant est prélevé puis filtré. Un dopage de CLD à 20 µg.kg^{- 1} est effectué dans le filtrat.

Le MIP (35 mg), contenu dans une cartouche, obtenu selon l'exemple 1 a été conditionné par passage d'1 ml d'hexane (étape 0 de conditionnement). Ensuite, une solution d'1 mL d'extrait de saumon dans l'acétonitrile dopée à chlordécone (CLD) a été percolée sur le MIP, par passage de ladite solution sur la cartouche (étape 1 de percolation).

Le MIP a ensuite été lavé avec 300 µL d'heptane (L1), séché sous air avec environ 20 mL, et ensuite lavé avec 1 mL d'un mélange acétonitrile/méthanol, 95/5, v/v (étape 2 de lavage). Enfin, le chlordécone a été élué par passage de 3 mL de méthanol (étape 3 d'élution).

La même procédure a été mise en oeuvre sur un NIP, obtenu selon l'exemple 2.

La procédure globale, à savoir l'extraction LLE, l'étape 0 de conditionnement, l'étape 1 de percolation, l'étape 2 de lavage, l'étape 3 d'élution et l'étape 4 de détection a été réalisée 3 fois sur le MIP, et sur le NIP. Les expériences ont été réalisées sur 3 cartouches issues de 3 lots de MIP et de NIP différents. Pour éviter tout biais, le même volume de solvant est percolé à travers le support de contrôle (NIP).

Pour chacune des expériences, seule la fraction d'élution (E), récupérée en bas des cartouches, a été analysée par la procédure LC/MS. Ainsi, la quantité de chlordécone présent dans la fraction d'élution a été déterminée et la moyenne des résultats a été calculée sur les 3 expériences (MIP et NIP).

La **Figure 8** montre les résultats obtenus pour le chlordécone dans une matrice alimentaire de **saumon extraite dans l'acétonitrile.** L'étape d'élution permet ensuite de récupérer le chlordécone retenu sur le MIP avec un rendement de CLD estimé dans l'éluât de 143%. **Ces résultats montrent que le MIP a la capacité de retenir le chlordécone dans un extrait d'échantillon alimentaire de saumon et de le restituer par élution,** par rapport au NIP qui ne retient pas ce produit (7%).

La sélectivité est maintenue en milieu réel avec un extrait de saumon dans l'acétonitrile.

Au vue de la complexité de l'échantillon (échantillon riche en lipide et en protéines)), des rendements supérieurs à 100% sont observés. Un effet de matrice lors de l'ionisation par electrospray au cours de l'analyse LC/MS tend à surestimer les concentrations mesurées

### Exemple 9 - Purification/détection d'un échantillon alimentaire de tomate dans l'acétonitrile

Le même protocole que celui l'exemple 8 a été réalisé avec comme échantillon alimentaire une matrice de tomate.

La **Figure 9** montre les résultats obtenus pour le chlordécone dans une matrice alimentaire de **tomate extraite dans l'acétonitrile.** L'étape d'élution permet ensuite de récupérer le chlordécone retenu sur le MIP avec un rendement de CLD estimé dans l'éluât de 157 %. **Ces résultats montrent que le MIP a la capacité de retenir le chlordécone dans un extrait d'échantillon alimentaire de tomate et de le restituer par élution,** par rapport au NIP qui ne retient pas ce produit (10%).

La sélectivité du MIP est maintenue en milieu réel avec un extrait de tomate dans l'acétonitrile.

## Revendications

1. Polymère à empreintes moléculaires (MIP) dans lequel le polymère est du poly-4-vinylpyridine, ou du poly-2-vinylpyridine, réticulé par un agent réticulant,
dans lequel l'agent réticulant est choisi parmi l'éthylène glycol diméthacrylate (EGDMA), le triméthylolpropane triméthacrylate (TRIM) et le pentaérythritol triacrylate (PETRA),
ledit polymère à empreintes moléculaires contenant des sites d'empreinte pour du chlordécol et/ou du chlordécone,
notamment dans lequel le polymère est du poly-4-vinylpyridine (4-VP MIP) réticulé par de l'éthylène glycol diméthacrylate (EGDMA).

2. Polymère à empreintes moléculaires selon la revendication 1, dans lequel le polymère est du poly-4-vinylpyridine, réticulé par un agent réticulant,
dans lequel l'agent réticulant est choisi parmi l'éthylène glycol diméthacrylate (EGDMA), le triméthylolpropane triméthacrylate (TRIM) et le pentaérythritol triacrylate (PETRA),
ledit polymère à empreintes moléculaires contenant des sites d'empreinte pour du chlordécol et/ou du chlordécone.

3. Polymère à empreintes moléculaires selon l'une des revendications 1 ou 2, ledit polymère à empreintes moléculaires possédant une capacité de retenir du chlordécone, déterminée par LC/MS, à raison de 36 µg par gramme de polymère.

4. Procédé de préparation d'un polymère à empreintes moléculaires, dans lequel le procédé comprend :
• une étape A de polymérisation d'un monomère et d'un agent de réticulation, ladite étape A de polymérisation étant effectuée en présence d'un porogène, d'un initiateur de polymérisation radicalaire, et du chlordécol,
dans lequel le monomère est choisi parmi le 4-vinylpyridine et le 2-vinylpyridine,
dans lequel l'agent de réticulation est choisi parmi l'éthylène glycol diméthacrylate (EGDMA), le triméthylolpropane triméthacrylate (TRIM) et le pentaérythritol triacrylate (PETRA),
dans lequel le porogène est de l'acétonitrile ou du dichlorométhane, et
dans lequel l'initiateur de polymérisation radicalaire est notamment de l'AIBN,
pour obtenir un polymère à empreintes moléculaires contenant du chlordécol.

5. Procédé de préparation selon la revendication 4, comprenant en outre :
après l'étape A de polymérisation, une étape B d'élimination du chlordécol du polymère à empreintes moléculaires, notamment par lavage, pour obtenir ledit polymère dépourvu de chlordécol, ladite étape B d'élimination étant notamment effectuée avec du méthanol,
et optionnellement,
entre l'étape A de polymérisation, et l'étape B d'élimination du chlordécol, une étape C de broyage, pour obtenir un polymère à empreintes moléculaires broyé,
et optionnellement,
après l'étape C de broyage, une étape D de tamisage, pour obtenir un polymère à empreintes moléculaires tamisé, ayant notamment une granulométrie comprise de 25 à 40 µm,
et optionnellement,
après l'étape D de tamisage, une étape E de sédimentation, pour obtenir un polymère à empreintes moléculaires sédimenté, ayant notamment une granulométrie comprise de 25 à 40 µm, ladite étape E de sédimentation étant notamment effectuée avec un mélange méthanol/eau, en particulier dans un ratio 80/20,
de préférence dans lequel le monomère est le 4-vinylpyridine, et le porogène est de l'acétonitrile.

6. Procédé de préparation selon l'une des revendications 4 à 5, dans lequel :
• le ratio molaire monomère : agent de réticulation utilisé dans l'étape A de polymérisation est compris de 3 : 30, de préférence d'environ 4 : 20, et/ou
• le ratio molaire chlordécol : monomère utilisé dans l'étape A de polymérisation est compris de 0,75 : 6, de préférence d'environ 1 : 4, et/ou
• le ratio molaire chlordécol : agent de réticulation utilisé dans l'étape A de polymérisation est compris de 0,5 : 30, de préférence d'environ 1 : 20,
notamment, dans lequel le ratio chlordécol : monomère : agent de réticulation utilisé dans l'étape A de polymérisation est d'environ 1 : 4 : 20,
en particulier dans lequel l'étape A de polymérisation est effectuée :
soit à une température comprise de 50 à 54 °C, notamment à une température de 54 °C, le porogène étant de l'acétonitrile,
soit à une température comprise de 25 à 30 °C, le porogène étant du dichlorométhane.

7. Procédé de préparation selon l'une des revendications 4 à 6, comprenant :
• une étape A de polymérisation du 4-vinylpyridine et l'éthylène glycol diméthacrylate, dans de l'acétonitrile, en présence d'AIBN et de chlordécol, pour obtenir un polymère à empreintes moléculaires contenant du chlordécol,
• une étape C de broyage, pour obtenir un polymère à empreintes moléculaires broyé,
• une étape D de tamisage, pour obtenir un polymère à empreintes moléculaires tamisé, ayant notamment une granulométrie inférieure à 40 µm,
• une étape E de sédimentation, pour obtenir un polymère à empreintes moléculaires sédimenté, ayant notamment une granulométrie comprise de 25 à 40 µm, et
• optionnellement une étape B d'élimination du chlordécol du polymère à empreintes moléculaires, notamment par du méthanol, pour obtenir ledit polymère à empreintes moléculaires dépourvu de chlordécol.

8. Polymère à empreintes moléculaires susceptible d'être obtenu par le procédé selon l'une des revendications 4 à 7.

9. Utilisation d'un polymère imprimé selon l'une des revendications 1, 2, 3 ou 8, pour la purification d'un échantillon comprenant le chlordécol et/ou du chlordécone,
dans laquelle l'échantillon est notamment un échantillon de sol, un échantillon aqueux, un échantillon alimentaire ou un échantillon biologique.

10. Cartouche comprenant un polymère à empreintes moléculaires selon l'une des revendications 1, 2,3 ou 8.

11. Procédé de purification d'un échantillon comprenant du chlordécone et/ou du chlordécol, lequel procédé comprenant :
• une étape 1 de percolation dudit échantillon sur un polymère à empreintes moléculaires, selon l'une des revendications, 1, 2, 3 ou 8, en particulier le 4-VP MIP, pour obtenir un polymère à empreintes moléculaires sur lequel le chlordécone et/ou le chlordécol est retenu,
dans lequel l'échantillon à percoler est notamment un échantillon aqueux, un échantillon alimentaire, un échantillon de sol ou un échantillon biologique,
ladite étape 1 de percolation est notamment effectuée avec de l'acétonitrile ou de l'hexane ou de l'heptane.

12. Procédé de purification selon la revendication 11, comprenant en outre :
• avant l'étape 1 de percolation, une étape 0 de conditionnement, du polymère à empreintes moléculaires, en particulier le 4-VP MIP, pour obtenir un polymère à empreintes moléculaires conditionné,
ladite étape 0 de conditionnement étant notamment effectuée avec de l'acétonitrile ou de l'hexane ou de l'heptane,
et/ou
• après l'étape 1 de percolation, une étape 2 de lavage du polymère à empreintes moléculaires sur lequel le chlordécone et/ou le chlordécol est percolé, pour obtenir un polymère à empreintes moléculaires lavé,
ladite étape 2 de lavage étant notamment effectuée avec un mélange acétonitrile/méthanol, notamment dans un ratio 95/5,
et optionnellement,
• après l'étape 2 de lavage, une étape 3 d'élution du chlordécone, pour obtenir un éluât comprenant le chlordécone et/ou le chlordécol,
ladite étape 3 d'élution étant notamment effectuée avec du méthanol,
et optionnellement,
• après l'étape 3 d'élution, une étape 4 détection du chlordécone par analyse de l'éluât, notamment par LC/MS ou GC/MS.

13. Procédé de purification selon la revendication 11, comprenant en outre :
• avant l'étape 1 de percolation, une étape 0 de conditionnement, du polymère à empreintes moléculaires, en particulier le 4-VP MIP, pour obtenir un polymère à empreintes moléculaires conditionné,
ladite étape 0 de conditionnement étant notamment effectuée avec de l'acétonitrile ou de l'hexane ou de l'heptane,
et/ou
• après l'étape 1 de percolation, une étape 2 de lavage du polymère à empreintes moléculaires sur lequel le chlordécone et/ou le chlordécol est percolé, pour obtenir un polymère à empreintes moléculaires lavé,
ladite étape 2 de lavage étant notamment effectuée avec un premier lavage avec l'heptane suivi d'une étape de séchage et un deuxième lavage avec un mélange acétonitrile/méthanol, notamment dans un ratio 95/5,
et optionnellement,
• après l'étape 2 de lavage, une étape 3 d'élution du chlordécone, pour obtenir un éluât comprenant le chlordécone et/ou le chlordécol,
ladite étape 3 d'élution étant notamment effectuée avec du méthanol,
et optionnellement,
• après l'étape 3 d'élution, une étape 4 détection du chlordécone par analyse de l'éluât, notamment par LC/MS ou GC/MS.

14. Procédé de purification selon l'une des revendications 11 ou 12, comprenant les étapes suivantes :
• une étape 0 de conditionnement du polymère à empreintes moléculaires, en particulier le 4-VP MIP, notamment par de l'acétonitrile ou de l'hexane ou de l'heptane, pour obtenir un polymère à empreintes moléculaires conditionné,
• une étape 1 de percolation dudit échantillon sur ledit polymère à empreintes moléculaires conditionné, notamment dissous dans de l'acétonitrile ou de l'hexane ou de l'heptane, pour obtenir un polymère à empreintes moléculaires sur lequel le chlordécone et/ou le chlordécol est retenu,
• une étape 2 de lavage du polymère à empreintes moléculaires sur lequel le chlordécone et/ou le chlordécol est retenu, notamment par un mélange d'acétonitrile/méthanol 95/5, pour obtenir un polymère à empreintes moléculaires lavé, et
• une étape 3 d'élution du chlordécone et/ou du chlordécol, pour obtenir un éluât comprenant le chlordécone et/ou le chlordécol.

15. Procédé de purification selon l'une des revendications 11 ou 13, comprenant les étapes suivantes :
• une étape 0 de conditionnement du polymère à empreintes moléculaires, en particulier le 4-VP MIP, notamment par de l'acétonitrile ou de l'hexane ou de l'heptane, pour obtenir un polymère à empreintes moléculaires conditionné,
• une étape 1 de percolation dudit échantillon sur ledit polymère à empreintes moléculaires conditionné, notamment dissous dans de l'acétonitrile ou de l'hexane ou de l'heptane, pour obtenir un polymère à empreintes moléculaires sur lequel le chlordécone et/ou le chlordécol est retenu,
• une étape 2 de lavage du polymère à empreintes moléculaires sur lequel le chlordécone et/ou le chlordécol est retenu, notamment par un premier lavage avec l'heptane suivi d'une étape de séchage et un deuxième lavage avec un mélange acétonitrile/méthanol, notamment dans un ratio 95/5, pour obtenir un polymère à empreintes moléculaires lavé, et
une étape 3 d'élution du chlordécone et/ou du chlordécol, pour obtenir un éluât comprenant le chlordécone et/ou le chlordécol.

## Patentansprüche

1. Molekular geprägtes Polymer (MIP), wobei das Polymer Poly-4-vinylpyridin oder Poly-2-vinylpyridin ist, das mit einem Vernetzungsmittel vernetzt ist,
wobei das Vernetzungsmittel ausgewählt ist aus Ethylenglykoldimethacrylat (EGDMA), Trimethylolpropantrimethacrylat (TRIM) und Pentaerythritoltriacrylat (PETRA),
wobei das molekular geprägte Polymer Prägungsstellen für Chlordecol und/oder Chlordecon enthält,
wobei das Polymer insbesondere Poly-4-vinylpyridin (4-VP MIP) ist, das mit Ethylenglykoldimethacrylat (EGDMA) vernetzt ist.

2. Molekular geprägtes Polymer nach Anspruch 1, wobei das Polymer Poly-4-vinylpyridin ist, das mit einem Vernetzungsmittel vernetzt ist,
wobei das Vernetzungsmittel ausgewählt ist aus Ethylenglykoldimethacrylat (EGDMA), Trimethylolpropantrimethacrylat (TRIM) und Pentaerythritoltriacrylat (PETRA),
wobei das molekular geprägte Polymer Prägungsstellen für Chlordecol und/oder Chlordecon enthält.

3. Molekular geprägtes Polymer nach einem der Ansprüche 1 oder 2, wobei das molekular geprägte Polymer eine Fähigkeit zur Bindung von Chlordecon, bestimmt mittels LC/MS, in einer Menge von 36 µg pro Gramm Polymer besitzt.

4. Verfahren zur Herstellung eines molekular geprägten Polymers, wobei das Verfahren Folgendes umfasst:
• einen Schritt A zur Polymerisation eines Monomers und eines Vernetzungsmittels, wobei der Schritt A zur Polymerisation in Gegenwart eines Porogens, eines Initiators der radikalischen Polymerisation und des Chlordecols durchgeführt wird,
wobei das Monomer ausgewählt ist aus 4-Vinylpyridin und 2-Vinylpyridin,
wobei das Vernetzungsmittel ausgewählt ist aus Ethylenglykoldimethacrylat (EGDMA), Trimethylolpropantrimethacrylat (TRIM) und Pentaerythritoltriacrylat (PETRA),
wobei das Porogen Acetonitril oder Dichlormethan ist, und
wobei der Initiator der radikalischen Polymerisation insbesondere AIBN ist, um ein molekular geprägtes Polymer zu erhalten, das Chlordecol enthält.

5. Verfahren zur Herstellung nach Anspruch 4, ferner umfassend :
nach dem Schritt A zur Polymerisation einen Schritt B zur Entfernung von Chlordecol aus dem molekular geprägten Polymer, insbesondere durch Waschen, um dieses Polymer ohne Chlordecol zu erhalten, wobei der Schritt B zur Entfernung insbesondere mit Methanol durchgeführt wird,
und optional
zwischen Schritt A zur Polymerisation und Schritt B zur Entfernung des Chlordecols einen Schritt C zum Mahlen, um ein gemahlenes molekular geprägtes Polymer zu erhalten,
und optional,
nach dem Schritt C zum Mahlen einen Schritt D zum Sieben, um ein gesiebtes molekular geprägtes Polymer zu erhalten, das insbesondere eine Teilchengröße im Bereich zwischen 25 und 40 µm aufweist,
und optional,
nach dem Schritt D zum Sieben einen Schritt E zur Sedimentation, um ein sedimentiertes molekular geprägtes Polymer zu erhalten, das insbesondere eine Teilchengröße im Bereich zwischen 25 und 40 µm aufweist, wobei der Schritt E zur Sedimentation insbesondere mit einem Methanol/Wasser-Gemisch, insbesondere in einem Verhältnis von 80/20, durchgeführt wird,
vorzugsweise wobei das Monomer 4-Vinylpyridin ist und das Porogen Acetonitril ist.

6. Verfahren zur Herstellung nach einem der Ansprüche 4 bis 5, wobei:
• das in Schritt A zur Polymerisation verwendete Molverhältnis Monomer:Vernetzungsmittel im Bereich von 3:30, vorzugsweise von etwa 4:20 liegt, und/oder
• das in Schritt A zur Polymerisation verwendete Molverhältnis Chlordecol:Monomer im Bereich von 0,75:6, vorzugsweise von etwa 1:4 liegt und/oder
• das in Schritt A zur Polymerisation verwendete Molverhältnis Chlordecol:Vernetzungsmittel im Bereich von 0,5:30, vorzugsweise von etwa 1:20 liegt, wobei das in Schritt A zur Polymerisation verwendete Verhältnis Chlordecol:Monomer:Vernetzungsmittel insbesondere bei etwa 1:4:20 liegt,
wobei der Schritt A zur Polymerisation insbesondere durchgeführt wird:
entweder bei einer Temperatur im Bereich von 50 bis 54 °C, insbesondere bei einer Temperatur von 54 °C, wobei das Porogen Acetonitril ist,
oder bei einer Temperatur im Bereich von 25 bis 30 °C, wobei das Porogen Dichlormethan ist.

7. Verfahren zur Herstellung nach einem der Ansprüche 4 bis 6, umfassend :
• einen Schritt A zur Polymerisation von 4-Vinylpyridin und Ethylenglykoldimethacrylat in Acetonitril in Gegenwart von AIBN und Chlordecol, um ein molekular geprägtes Polymer zu erhalten, das Chlordecol enthält,
• einen Schritt C zum Mahlen, um ein gemahlenes molekular geprägtes Polymer zu erhalten,
• einen Schritt D zum Sieben, um ein gesiebtes molekular geprägtes Polymer zu erhalten, das insbesondere eine Teilchengröße von weniger als 40 µm aufweist,
• einen Schritt E zur Sedimentation, um ein sedimentiertes molekular geprägtes Polymer zu erhalten, das insbesondere eine Teilchengröße im Bereich von 25 bis 40 µm aufweist,
• optional einen Schritt B zum Entfernen von Chlordecol aus dem molekular geprägten Polymer, insbesondere durch Methanol, um das molekular geprägte Polymer frei von Chlordecol zu erhalten.

8. Molekular geprägtes Polymer, das durch das Verfahren nach einem der Ansprüche 4 bis 7 erhalten werden kann.

9. Verwendung eines geprägten Polymers nach einem der Ansprüche 1, 2, 3 oder 8 zur Reinigung einer Probe, die Chlordecol und/oder Chlordecon umfasst,
wobei die Probe insbesondere eine Bodenprobe, eine wässrige Probe, eine Lebensmittelprobe oder eine biologische Probe ist.

10. Kartusche, umfassend ein molekular geprägtes Polymer nach einem der Ansprüche 1, 2, 3 oder 8.

11. Verfahren zur Reinigung einer Probe, umfassend Chlordecon und/oder Chlordecol, wobei das Verfahren Folgendes umfasst:
• einen Schritt 1 zur Perkolation der Probe auf einem molekular geprägten Polymer nach einem der Ansprüche 1, 2, 3 oder 8, insbesondere 4-VP MIP, um ein molekular geprägtes Polymer zu erhalten, auf dem Chlordecon und/oder Chlordecol zurückgehalten wird,
wobei die zu perkolierende Probe insbesondere eine wässrige Probe, eine Lebensmittelprobe, eine Bodenprobe oder eine biologische Probe ist, wobei der Schritt 1 zur Perkolation insbesondere mit Acetonitril oder Hexan oder Heptan durchgeführt.

12. Verfahren zur Reinigung nach Anspruch 11, das ferner Folgendes umfasst:
• vor dem Schritt 1 zur Perkolation einen Schritt 0 zur Konditionierung des molekular geprägten Polymers, insbesondere des 4-VP MIP, um ein konditioniertes molekular geprägtes Polymer zu erhalten,
wobei der Schritt 0 zur Konditionierung insbesondere mit Acetonitril oder Hexan oder Heptan durchgeführt wird,
und/oder
• nach dem Schritt 1 zur Perkolation einen Schritt 2 zum Waschen des molekular geprägten Polymers, auf dem Chlordecon und/oder Chlordecol perkoliert wird, um ein gewaschenes molekular geprägtes Polymer zu erhalten,
wobei der Schritt 2 zum Waschen insbesondere mit einem Acetonitril/Methanol-Gemisch, insbesondere in einem Verhältnis von 95/5, durchgeführt wird,
und optional,
• nach dem Schritt 2 zum Waschen einen Schritt 3 zur Elution des Chlordecons, um ein Eluat zu erhalten, das Chlordecon und/oder Chlordecol umfasst,
wobei der Schritt 3 zur Elution insbesondere mit Methanol durchgeführt wird,
und optional,
• nach dem Schritt 3 zur Elution einen Schritt 4 zum Nachweis von Chlordecon durch Analyse des Eluats, insbesondere mittels LC/MS oder GC/MS.

13. Verfahren zur Reinigung nach Anspruch 11, das ferner Folgendes umfasst:
• vor dem Schritt 1 zur Perkolation einen Schritt 0 zur Konditionierung des molekular geprägten Polymers, insbesondere des 4-VP MIP, um ein konditioniertes molekular geprägtes Polymer zu erhalten,
wobei der Schritt 0 zur Konditionierung insbesondere mit Acetonitril oder Hexan oder Heptan durchgeführt wird,
und/oder
• nach dem Schritt 1 zur Perkolation einen Schritt 2 zum Waschen des molekular geprägten Polymers, auf dem Chlordecon und/oder Chlordecol perkoliert wird, um ein gewaschenes molekular geprägtes Polymer zu erhalten,
wobei der Schritt 2 zum Waschen insbesondere mit einer ersten Wäsche mit Heptan gefolgt von einem Schritt zur Trocknung und einer zweiten Wäsche mit einem Acetonitril/Methanol-Gemisch, insbesondere in einem Verhältnis von 95:5, durchgeführt wird,
und optional,
• nach dem Schritt 2 zum Waschen einen Schritt 3 zur Elution von Chlordecon, um ein Eluat zu erhalten, das Chlordecon und/oder Chlordecol enthält,
wobei dieser Schritt 3 zur Elution insbesondere mit Methanol durchgeführt wird,
und optional
• nach Schritt 3 zur Elution einen Schritt 4 zum Nachweis von Chlordecon durch Analyse des Eluats, insbesondere mittels LC/MS oder GC/MS.

14. Verfahren zur Reinigung nach einem der Ansprüche 11 oder 12, das die folgenden Schritte umfasst:
• einen Schritt 0 zur Konditionierung des molekular geprägten Polymers, insbesondere des 4-VP MIP, insbesondere mit Acetonitril oder Hexan oder Heptan, um ein konditioniertes molekular geprägtes Polymer zu erhalten,
• einen Schritt 1 zur Perkolation der Probe über das konditionierte molekular geprägte Polymer, insbesondere gelöst in Acetonitril oder Hexan oder Heptan, um ein molekular geprägtes Polymer zu erhalten, auf dem Chlordecon und/oder Chlordecol zurückgehalten wird,
• einen Schritt 2 zum Waschen des molekular geprägten Polymers, auf dem Chlordecon und/oder Chlordecol zurückgehalten wird, insbesondere mit einem 95/5-Gemisch aus Acetonitril/Methanol, um ein gewaschenes molekular geprägtes Polymer zu erhalten, und
• einen Schritt 3 zur Elution von Chlordecon und/oder Chlordecol, um ein Eluat zu erhalten, das Chlordecon und/oder Chlordecol enthält.

15. Verfahren zur Reinigung nach einem der Ansprüche 11 oder 13, das die folgenden Schritte umfasst:
• einen Schritt 0 zur Konditionierung des molekular geprägten Polymers, insbesondere des 4-VP MIP, insbesondere mit Acetonitril oder Hexan oder Heptan, um ein konditioniertes molekular geprägtes Polymer zu erhalten,
• einen Schritt 1 zur Perkolation der Probe auf dem konditionierten molekular geprägten Polymer, insbesondere gelöst in Acetonitril oder Hexan oder Heptan, um ein molekular geprägtes Polymer zu erhalten, auf dem Chlordecon und/oder Chlordecol zurückgehalten wird,
• einen Schritt 2 zum Waschen des molekular geprägten Polymers, auf dem Chlordecon und/oder Chlordecol zurückgehalten wird, insbesondere durch eine erste Wäsche mit Heptan, gefolgt von einem Schritt zur Trocknung, und eine zweite Wäsche mit einem Acetonitril/Methanol-Gemisch, insbesondere im Verhältnis 95:5, um ein gewaschenes molekular geprägtes Polymer zu erhalten, und
einen Schritt 3 der Elution von Chlordecon und/oder Chlordecol, um ein Eluat zu erhalten, das Chlordecon und/oder Chlordecol enthält.

## Claims

1. Molecularly imprinted polymer (MIP) wherein the polymer is poly-4-vinylpyridine, or poly-2-vinylpyridine, crosslinked by a crosslinking agent,
wherein the crosslinking agent is selected from ethylene glycol dimethacrylate (EGDMA), trimethylolpropane trimethacrylate (TRIM) and pentaerythritol triacrylate (PETRA),
said molecularly imprinted polymer containing imprint sites for chlordecol and/or chlordecone,
in particular wherein the polymer is poly-4-vinylpyridine (4-VP MIP) crosslinked with ethylene glycol dimethacrylate (EGDMA).

2. A molecularly imprinted polymer according to claim 1, wherein the polymer is poly-4-vinylpyridine, crosslinked by a crosslinking agent,
wherein the crosslinking agent is selected from ethylene glycol dimethacrylate (EGDMA), trimethylolpropane trimethacrylate (TRIM) and pentaerythritol triacrylate (PETRA),
said molecularly imprinted polymer containing imprint sites for chlordecol and/or chlordecone.

3. A molecularly imprinted polymer according to one of claims 1 or 2, said molecularly imprinted polymer having a chlordecone retention capacity, determined by LC/MS, at a rate of 36 µg per gram of polymer.

4. A method of preparation of a molecularly imprinted polymer, wherein the method comprises:
• a step A of polymerization of a monomer and a crosslinking agent,
said step A of polymerization being carried out in the presence of a porogen, a radical polymerization initiator, and chlordecol,
wherein the monomer is selected from 4-vinylpyridine and 2-vinylpyridine,
wherein the crosslinking agent is selected from ethylene glycol dimethacrylate (EGDMA), trimethylolpropane trimethacrylate (TRIM) and pentaerythritol triacrylate (PETRA),
wherein the porogen is acetonitrile or dichloromethane, and
wherein the radical polymerization initiator is in particular AIBN,
to obtain a molecularly imprinted polymer containing chlordecol.

5. The method of preparation according to claim 4, further comprising:
after the step A of polymerization, a step B of removal chlordecol from the molecularly imprinted polymer, in particular by washing, to obtain said polymer free of chlordecol, said step B of removing being carried out in particular with methanol,
and optionally
between the step A of polymerization and the step B of removal chlordecol, a grinding step C, to obtain a ground molecularly imprinted polymer,
and optionally
after the grinding step C, a sieving step D, to obtain a sieved molecularly imprinted polymer, in particular having a particle size from 25 to 40 µm,
and optionally
after the sieving step D, a sedimentation step E, to obtain a sedimented molecularly imprinted polymer, in particular having a particle size from 25 to 40 µm, said sedimentation step E being carried out in particular with a methanol/water mixture, in particular in a ratio 80/20,
preferably wherein the monomer is 4-vinylpyridine and the porogen is acetonitrile.

6. The method of preparation according to one of claims 4 to 5, wherein :
• the molar ratio of monomer : crosslinking agent used in the step A of polymerization is in the range 3 : 30, preferably about 4 : 20, and/or
• the molar ratio of chlordecol : monomer used in the step A of polymerization is 0.75 : 6, preferably about 1 : 4, and/or
• the molar ratio of chlordecol : crosslinking agent used in the step A of polymerization is in the range 0.5 : 30, preferably about 1 : 20,
in particular, wherein the ratio of chlordecol : monomer : crosslinking agent used in the step A pf polymerization is about 1 : 4 : 20,
in particular wherein the step A of polymerization is carried out :
either at a temperature from 50 to 54°C, in particular at 54°C, the porogen being acetonitrile,
or at a temperature from 25 to 30°C, the porogen being dichloromethane.

7. The method of preparation according to any one of claims 4 to 6, comprising :
• a step A of polymerization of 4-vinylpyridine and ethylene glycol dimethacrylate, in acetonitrile, in the presence of AIBN and chlordecol, to obtain a molecularly imprinted polymer containing chlordecol,
• a grinding step C, to obtain a ground molecularly imprinted polymer,
• a sieving step D, to obtain a sieved molecularly imprinted polymer, in particular having a particle size of less than 40 µm,
• a sedimentation step E, to obtain a sedimented molecularly imprinted polymer, in particular having a particle size of 25 to 40 µm, and
• optionally, a step B of removal chlordecol from the molecularly imprinted polymer, in particular using methanol, to obtain said molecularly imprinted polymer free of chlordecol.

8. Molecularly imprinted polymer obtainable by the method according to any one of claims 4 to 7.

9. Use of an imprinted polymer according to any one of claims 1, 2, 3 or 8, for the purification of a sample comprising chlordecol and/or chlordecone,
wherein the sample is in particular a soil sample, an aqueous sample, a food sample or a biological sample.

10. A cartridge comprising a molecularly imprinted polymer according to any one of claims 1, 2, 3 or 8.

11. A method of purification of a sample comprising chlordecone and/or chlordecol, said method comprises:
• a step 1 of percolation of said sample on a molecularly imprinted polymer, according to one of claims 1, 2, 3 or 8, in particular 4-VP MIP, to obtain a molecularly imprinted polymer on which chlordecone and/or chlordecol is retained,
wherein the sample to be percolated is in particular an aqueous sample, a food sample, a soil sample or a biological sample,
said step 1 of percolation is carried out using acetonitrile, hexane or heptane.

12. The method of purification according to claim 11, further comprising:
• prior to the step 1 of percolation, a step 0 of conditioning, of the molecularly imprinted polymer, in particular 4-VP MIP, to obtain a conditioned molecularly imprinted polymer, said step 0 of conditioning being carried out in particular with acetonitrile or hexane or heptane,
and/or
• after the step 1 of percolation, a step 2 of washing the molecularly imprinted polymer onto which the chlordecone and/or chlordecol has been percolated, to obtain a washed molecularly imprinted polymer,
said step 2 of washing being carried out in particular with an acetonitrile/methanol mixture, in particular in a 95/5 ratio,
and optionally
• after the step 2 of washing, a step 3 of elution of chlordecone, to obtain an eluate comprising chlordecone and/or chlordecol,
said step 3 of elution being carried out in particular with methanol,
and optionally
• after the step 3 of elution, a step 4 of chlordecone detection by analysis of the eluate, in particular by LC/MS or GC/MS.

13. The method of purification according to claim 11, further comprising:
• prior to the step 1 of percolation, a step 0 of conditioning, of the molecularly imprinted polymer, in particular 4-VP MIP, to obtain a conditioned molecularly imprinted polymer, said step 0 of conditioning being carried out in particular with acetonitrile or hexane or heptane,
and/or
• after the step 1 of percolation, a step 2 of washing the molecularly imprinted polymer onto which the chlordecone and/or chlordecol has been percolated, to obtain a washed molecularly imprinted polymer,
said step 2 of washing being carried out in particular with a first wash with heptane followed by a drying step and a second wash with an acetonitrile/methanol mixture, in particular in a ratio 95/5,
and optionally
• after the step 2 of washing, a step 3 of elution of chlordecone, to obtain an eluate comprising chlordecone and/or chlordecol,
said step 3 of elution being carried out in particular with methanol,
and optionally
• after the elution step 3, a chlordecone detection step 4 by analysis of the eluate, in particular by LC/MS or GC/MS.

14. The method of purification according to one of claims 11 or 12, comprising the following steps:
• a step 0 of conditioning the molecularly imprinted polymer, in particular 4-VP MIP, in particular with acetonitrile or hexane or heptane, to obtain a conditioned molecularly imprinted polymer,
• a step 1 of percolation of said sample onto said conditioned molecularly imprinted polymer, notably dissolved in acetonitrile or hexane or heptane, to obtain a molecularly imprinted polymer on which chlordecone and/or chlordecol is retained,
• a step 2 of washing the molecularly imprinted polymer on which the chlordecone and/or chlordecol is retained, in particular with an acetonitrile/methanol 95/5 mixture, to obtain a washed molecularly imprinted polymer, and
• a step 3 of elution of chlordecone and/or chlordecol, to obtain an eluate comprising chlordecone and/or chlordecol.

15. Methode of purification according to any one of claims 11 or 13, comprising the following steps:
• a step 0 of conditioning the molecularly imprinted polymer, in particular 4-VP MIP, in particular with acetonitrile or hexane or heptane, to obtain a conditioned molecularly imprinted polymer,
• a step 1 of percolation of said sample onto said conditioned molecularly imprinted polymer, notably dissolved in acetonitrile or hexane or heptane, to obtain a molecularly imprinted polymer on which chlordecone and/or chlordecol is retained,
• a step 2 of washing the molecularly imprinted polymer on which the chlordecone and/or chlordecol is retained, in particular by a first wash with heptane followed by a drying step and a second wash with an acetonitrile/methanol mixture, in particular in a 95/5 ratio, to obtain a washed molecularly imprinted polymer, and
• a step 3 of elution of chlordecone and/or chlordecol, to obtain an eluate comprising chlordecone and/or chlordecol.
